# EUROPEAN PATENT SPECIFICATION

(11) **EP 3 628 335 B1**
(45) Date of publication and mention of the grant of the patent: **08.11.2023**
(21) Application number: 19206348.5
(22) Date of filing: 06.12.2013
(51) Int. Cl.: A61K 48/00

(54) **LIPIDIC NANOPARTICLES FOR MRNA DELIVERY IN THE LUNGS**
LIPIDNANOPARTIKEL ZUR LIEFERUG VON MRNA IN DEN LUNGEN
NANOPARTICULES LIPIDIQUES POUR L'ADMINISTRATION D'ARNM DANS LES POUMONS

(30) Priority: 07.12.2012 US 201261734753 P
(43) Date of publication of application: 01.04.2020
(62) Divisional of application: 13812359.1
(73) Proprietor: Translate Bio, Inc., Waltham, MA 02451 (US)
(72) Inventor: DEROSA, Frank, Lexington, MA 02421 (US); HEARTLEIN, Michael, Lexington, MA 02421 (US); GUILD, Braydon, Charles, Lexington, MA 02421 (US)
(74) Representative: Carpmaels & Ransford LLP

(56) References cited:
- WO-A1-2011/068810
- WO-A1-2013/185069
- WO-A1-2022/225918
- US-A- 5 885 613
- GEORGE C.L.S. ET AL.: "Surfactant-associated protein A provides critical immunoprotection in neonatal mice.", INFECTION AND IMMUNITY, vol. 76, no. 1, January 2008 (2008-01), pages 380-390, XP002797619,
- HAMVAS A., COLE F.S. & NOGEE L.M.: "Genetic disorders of surfactant proteins", NEONATOLOGY, vol. 91, 7 June 2007 (2007-06-07), pages 311-317, XP002797620,
- YIN X. ET AL.: "Correlation between surfactant protein B mRNA expression and neonatal respiratory distress syndrome", EXPERIMENTAL AND THERAPEUTIC MEDICINE, vol. 4, 17 August 2012 (2012-08-17), pages 815-819, XP002797621,
- KLIBANOV A L ET AL: "AMPHIPATHIC POLYETHYLENEGLYCOLS EFFECTIVELY PROLONG THE CIRCULATION TIME OF LIPOSOMES", FEBS LETTERS, ELSEVIER, AMSTERDAM, NL, vol. 268, no. 1, 1 July 1990 (1990-07-01), pages 235-237, XP001073450, ISSN: 0014-5793, DOI: 10.1016/0014-5793(90)81016-H

## Description

### BACKGROUND

Novel approaches and therapies are still needed for the treatment of protein deficiencies. Therapies, such as gene therapy, that increase the level or production of an affected protein or enzyme could provide a treatment or even a cure for such disorders. However, there have been several limitations to using conventional gene therapy for this purpose.

Conventional gene therapy involves the use of DNA for insertion of desired genetic information into host cells. The DNA introduced into the cell is usually integrated to a certain extent into the genome of one or more transfected cells, allowing for long-lasting action of the introduced genetic material in the host. While there may be substantial benefits to such sustained action, integration of exogenous DNA into a host genome may also have many deleterious effects. For example, it is possible that the introduced DNA will be inserted into an intact gene, resulting in a mutation which impedes or even totally eliminates the function of the endogenous gene. Thus, gene therapy with DNA may result in the impairment of a vital genetic function in the treated host, such as e.g., elimination or deleteriously reduced production of an essential enzyme or interruption of a gene critical for the regulation of cell growth, resulting in unregulated or cancerous cell proliferation. In addition, with conventional DNA based gene therapy it is necessary for effective expression of the desired gene product to include a strong promoter sequence, which again may lead to undesirable changes in the regulation of normal gene expression in the cell. It is also possible that the DNA based genetic material will result in the induction of undesired anti-DNA antibodies, which in turn, may trigger a possibly fatal immune response. Gene therapy approaches using viral vectors can also result in an adverse immune response. In some circumstances, the viral vector may even integrate into the host genome. In addition, production of clinical grade viral vectors is also expensive and time consuming. Targeting delivery of the introduced genetic material using viral vectors can also be difficult to control. Thus, while DNA based gene therapy has been evaluated for delivery of secreted proteins using viral vectors (US Patent No. 6,066,626; US2004/0110709; Amalfitano, A., et al., PNAS (1999) vol. 96, pp. 8861-66), these approaches may be limited for these various reasons.

Another obstacle apparent in these prior approaches at delivery of nucleic acids encoding secreted proteins, is in the levels of protein that are ultimately produced. It is difficult to achieve significant levels of the desired protein in the blood, and the amounts are not sustained over time. For example, the amount of protein produced by nucleic acid delivery does not reach normal physiological levels. S*ee e.g.,* US2004/0110709.

In contrast to DNA, the use of RNA as a gene therapy agent is substantially safer because (1) RNA does not involve the risk of being stably integrated into the genome of the transfected cell, thus eliminating the concern that the introduced genetic material will disrupt the normal functioning of an essential gene, or cause a mutation that results in deleterious or oncogenic effects; (2) extraneous promoter sequences are not required for effective translation of the encoded protein, again avoiding possible deleterious side effects; (3) in contrast to plasmid DNA (pDNA), messenger RNA (mRNA) is devoid of immunogenic CpG motifs so that anti-RNA antibodies are not generated; and (4) any deleterious effects that do result from mRNA based on gene therapy would be of limited duration due to the relatively short half-life of RNA. In addition, it is not necessary for mRNA to enter the nucleus to perform its function, while DNA must overcome this major barrier.

One reason that mRNA based gene therapy has not been used more in the past is that mRNA is far less stable than DNA, especially when it reaches the cytoplasm of a cell and is exposed to degrading enzymes. The presence of a hydroxyl group on the second carbon of the sugar moiety in mRNA causes steric hindrance that prevents the mRNA from forming the more stable double helix structure of DNA and thus makes the mRNA more prone to hydrolytic degradation. As a result, until recently, it was widely believed that mRNA was too labile to withstand transfection protocols. Advances in RNA stabilizing modifications have sparked more interest in the use of mRNA in place of plasmid DNA in gene therapy. Certain delivery vehicles, such as cationic lipid or polymer delivery vehicles may also help protect the transfected mRNA from endogenous RNases. Yet, in spite of increased stability of modified mRNA, delivery of mRNA to cells *in vivo* in a manner allowing for therapeutic levels of protein production is still a challenge, particularly for mRNA encoding full length proteins. While delivery of mRNA encoding secreted proteins has been contemplated (US2009/0286852), the levels of a full length secreted protein that would actually be produced via *in vivo* mRNA delivery are not known and there is not a reason to expect the levels would exceed those observed with DNA based gene therapy.

To date, significant progress using mRNA gene therapy has only been made in applications for which low levels of translation has not been a limiting factor, such as immunization with mRNA encoding antigens. Clinical trials involving vaccination against tumor antigens by intradermal injection of naked or protamine-complexed mRNA have demonstrated feasibility, lack of toxicity, and promising results. X. Su et al., Mol. Pharmaceutics 8:774-787 (2011). Unfortunately, low levels of translation has greatly restricted the exploitation of mRNA based gene therapy in other applications which require higher levels of sustained expression of the mRNA encoded protein to exert a biological or therapeutic effect.

WO 2011/068810 discloses compositions and methods of modulating the expression of a gene or the production of a protein by transfecting target cells with nucleic acids. The disclosed compositions demonstrate a high transfection efficiency and are capable of ameliorating diseases associated with protein or enzyme deficiencies.

WO 2013/185069 discloses compositions comprising mRNA formulated for pulmonary administration and related methods for the delivery of the mRNA and/or the mRNA-encoded protein to a non-lung cell or tissue. The compositions and methods may be used to prevent of ameliorate the symptoms of diseases associated with the mRNA-encoded protein.

The pathological consequences of deficiencies of surfactant proteins are described inter alia in George (2008) Infl. Immun. 76(1):380-390; Hamvas (2007) Neonatology 91:311-317; Zin (2012) Exp. Ther. Med. 4:815-819.

### SUMMARY

The invention as defined in the appended claims provides a composition for use in a method of treating a disease in a subject that is due to a deficiency of a pulmonary surfactant-associated protein, wherein the pulmonary surfactant-associated protein is selected from SFTPA1, SFTPA2, SFTPB, SFTPC, and SFTPD, wherein said method comprises pulmonary administration of the composition to the subject, said composition comprising: (a) at least one mRNA molecule at least a portion of which encodes the pulmonary surfactant-associated protein; and (b) a transfer vehicle comprising a lipid nanoparticle, wherein the lipid nanoparticle comprises one or more cationic lipids, one or more non-cationic lipids and one of more PEG-modified lipids.

In some embodiments, the mRNA can comprise one or more modifications that confer stability to the mRNA (e.g., compared to a wild-type or native version of the mRNA) and may also comprise one or more modifications relative to the wild-type which correct a defect implicated in the associated aberrant expression of the protein. For example, the nucleic acids of the present invention may comprise modifications to one or both of the 5' and 3' untranslated regions. Such modifications may include, but are not limited to, the inclusion of a partial sequence of a cytomegalovirus (CMV) immediate-early 1 (IE1) gene, a poly A tail, a Cap1 structure or a sequence encoding human growth hormone (hGH)). In some embodiments, the mRNA is modified to decrease mRNA immunogenicity.

The transfer vehicle may comprise at least one of the following cationic lipids: XTC (2,2-Dilinoleyl-4-dimethylaminoethyl-[1,3]-dioxolane) and MC3 (((6Z,9Z,28Z,31Z)-heptatriaconta-6,9,28,31-tetraen-19-yl 4-(dimethylamino)butanoate), ALNY-100 ((3aR,5s,6aS)-N,N-dimethyl-2,2-di((9Z,12Z)-octadeca-9,12-dienyl)tetrahydro-3aH-cyclopenta[d] [1 ,3]dioxol-5-amine)), NC98-5 (4,7,13-tris(3-oxo-3-(undecylamino)propyl)-N1,N16-diundecyl-4,7,10,13-tetraazahexadecane-1,16-diamide), C12-200, DLin-KC2-DMA, DODAP, HGT4003, ICE, HGT5000, or HGT5001(cis or *trans).* In embodiments, the transfer vehicle comprises cholesterol (chol). In some embodiments, the transfer vehicles comprises DMG-PEG2K. In certain embodiments, the transfer vehicle comprises one of the following lipid formulations:
C12-200, DOPE, chol, DMG-PEG2K;
DODAP, DOPE, cholesterol, DMG-PEG2K;
HGT5000, DOPE, chol, DMG-PEG2K;
HGT5001, DOPE, chol, DMG-PEG2K;
XTC, DSPC, chol, PEG-DMG;
MC3, DSPC, chol, PEG-DMG;
ALNY-100, DSPC, chol, PEG-DSG

In embodiments, the pulmonary surfactant-associated protein is produced by the target cell for sustained amounts of time. For example, the pulmonary surfactant-associated protein may be produced for more than one hour, more than four, more than six, more than 12, more than 24, more than 48 hours, or more than 72 hours after administration. In some embodiments the pulmonary surfactant-associated protein is expressed at a peak level about six hours after administration. In some embodiments the expression of the pulmonary surfactant-associated protein is sustained at least at a therapeutic level. In some embodiments the pulmonary surfactant-associated protein is expressed at at least a therapeutic level for more than one, more than four, more than six, more than 12, more than 24, more than 48 hours, or more than 72 hours after administration. In some embodiments the pulmonary surfactant-associated protein is detectable at the level in patient lung tissue. In some embodiments, the level of detectable pulmonary surfactant-associated protein is from continuous expression from the mRNA composition over periods of time of more than one, more than four, more than six, more than 12, more than 24, more than 48 hours, or more than 72 hours after administration.

In certain embodiments, the pulmonary surfactant-associated protein is produced at levels above normal physiological levels. The level of pulmonary surfactant-associated protein may be increased as compared to a control. In some embodiments, the control is the baseline physiological level of the pulmonary surfactant-associated protein in a normal individual or in a population of normal individuals. In other embodiments, the control is the baseline physiological level of the pulmonary surfactant-associated protein in an individual having a deficiency in the the pulmonary surfactant-associated protein or in a population of individuals having a deficiency in the the pulmonary surfactant-associated protein. In some embodiments, the control can be the normal level of the relevant pulmonary surfactant-associated protein in the individual to whom the composition is administered.

In certain embodiments the pulmonary surfactant-associated protein is expressed by the target cell at a level which is at least 1.5-fold, at least 2-fold, at least 5-fold, at least 10-fold, at least 20-fold, 30-fold, at least 100-fold, at least 500-fold, at least 5000-fold, at least 50,000-fold or at least 100,000-fold greater than a control. In some embodiments, the fold increase of expression greater than control is sustained for more than one, more than four, more than six, more than 12, more than 24, or more than 48 hours, or more than 72 hours after administration. In certain embodiments, the levels of pulmonary surfactant-associated protein are detectable at 3 days, 4 days, 5 days, or 1 week or more after administration.

In some embodiments administration comprises a single or repeated doses.

Certain embodiments relate to compositions that provide to a cell or subject mRNA, at least a part of which encodes a functional pulmonary surfactant-associated protein, in an amount that is substantially less that the amount of corresponding functional protein generated from that mRNA. Put another way, in certain embodiments the mRNA delivered to the cell can produce an amount of a pulmonary surfactant-associated protein that is substantially greater than the amount of mRNA delivered to the cell. For example, in a given amount of time, for example 1, 2, 3, 4, 5, 6, 7, 8, 9, 10, 12, 15, 20, or 24 hours from administration of the mRNA to a cell or subject, the amount of corresponding pulmonary surfactant-associated protein generated by that mRNA can be at least 1.5, 2, 3, 5, 10, 15, 20, 25, 50, 100, 150, 200, 250, 300, 400, 500, or more times greater than the amount of mRNA actually administered to the cell or subject. This can be measured on a mass-by-mass basis, on a mole-by-mole basis, and/or on a molecule-by-molecule basis. The pulmonary surfactant-associated protein can be measured in various ways. For a subject, the measured protein can be protein measured in the lung. Moreover, a baseline amount of endogenous protein can be measured in the subject prior to administration of the mRNA and then subtracted from the protein measured after administration of the mRNA to yield the amount of corresponding protein generated from the mRNA. In this way, the mRNA can provide a reservoir or depot source of a large amount of therapeutic material to the cell or subject, for example, as compared to amount of mRNA delivered to the cell or subject. The depot source can act as a continuous source for polypeptide expression from the mRNA over sustained periods of time.

The above discussed and many other features and attendant advantages of the present invention will become better understood by reference to the following detailed description of the invention when taken in conjunction with the accompanying examples. The various embodiments described herein are complimentary and can be combined or used together in a manner understood by the skilled person in view of the teachings contained herein.

### BRIEF DESCRIPTION OF THE DRAWINGS

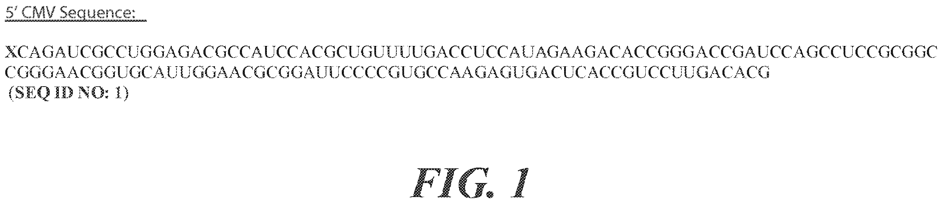

**FIG. 1** shows the nucleotide sequence of a 5' CMV sequence (SEQ ID NO: 1), wherein X, if present is GGA.

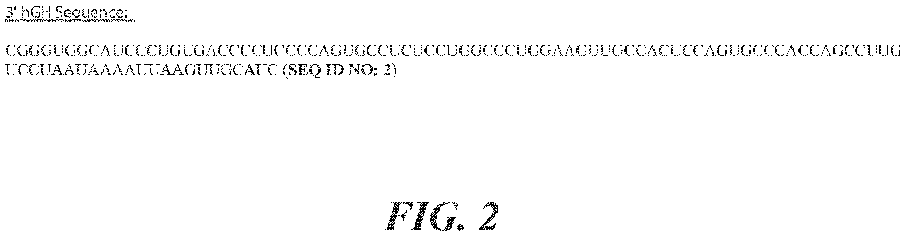

**FIG. 2** shows the nucleotide sequence of a 3' hGH sequence (SEQ ID NO:2).

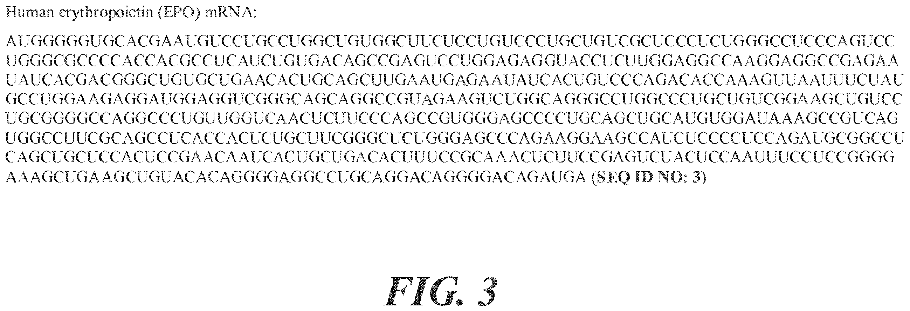

**FIG. 3** shows the nucleotide sequence of human erythropoietin (EPO) mRNA (SEQ ID NO:3). This sequence can be flanked on the 5' end with SEQ ID NO: 1 and on the 3' end with SEQ ID NO:2.

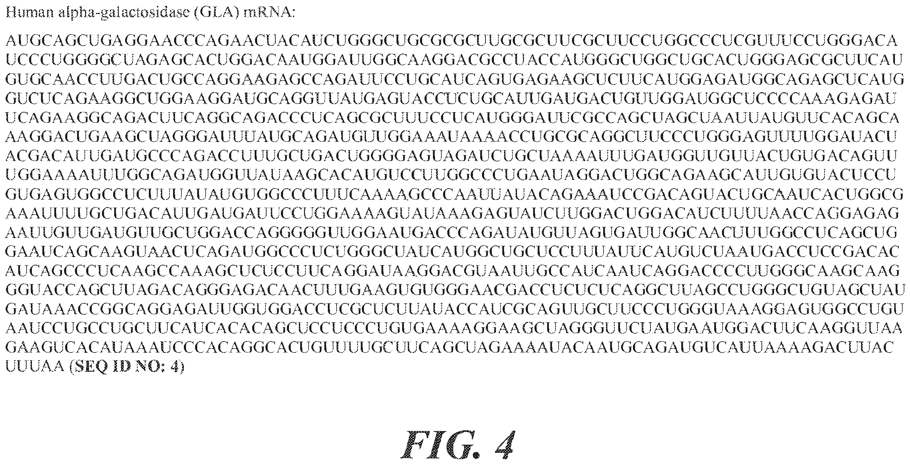

**FIG. 4** shows the nucleotide sequence of human alpha-galactosidase (GLA) mRNA (SEQ ID NO:4). This sequence can be flanked on the 5' end with SEQ ID NO: 1 and on the 3' end with SEQ ID NO:2.

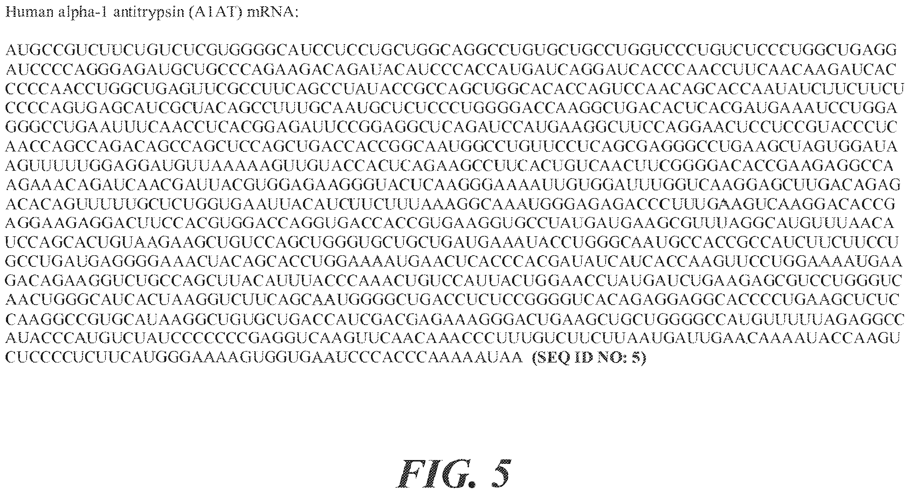

**FIG. 5** shows the nucleotide sequence of human alpha-1 antitrypsin (A1AT) mRNA (SEQ ID NO:5). This sequence can be flanked on the 5' end with SEQ ID NO: 1 and on the 3' end with SEQ ID NO:2.

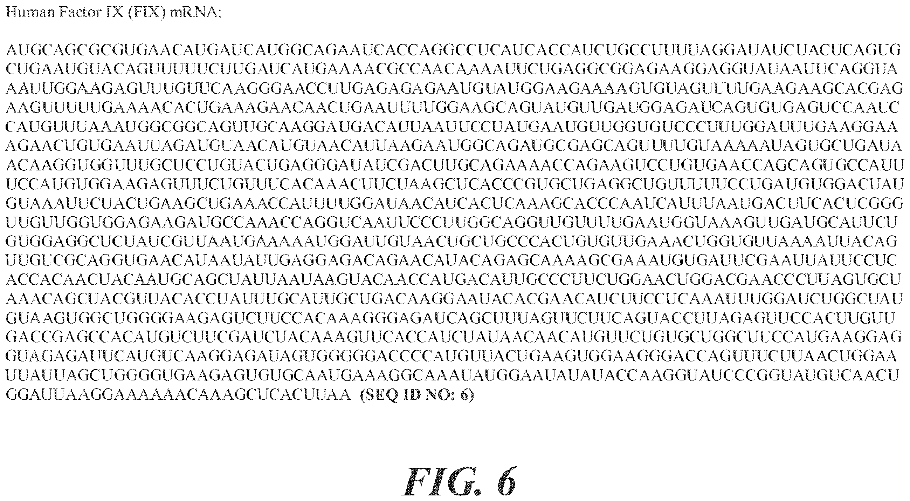

**FIG. 6** shows the nucleotide sequence of human factor IX (FIX) mRNA (SEQ ID NO:6). This sequence can be flanked on the 5' end with SEQ ID NO: 1 and on the 3' end with SEQ ID NO:2.

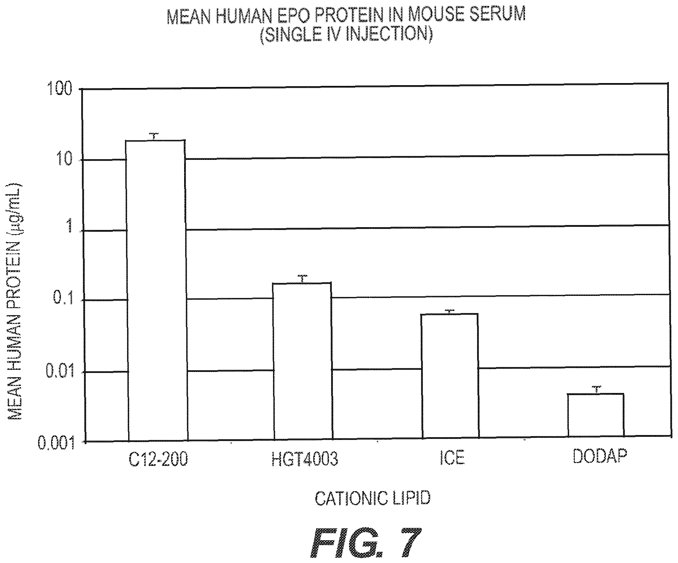

**FIG. 7** shows quantification of secreted hEPO protein levels as measured via ELISA. The protein detected is a result of its production from hEPO mRNA delivered intravenously via a single dose of various lipid nanoparticle formulations. The formulations C12-200 (30 ug), HGT4003 (150 ug), ICE (100 ug), DODAP (200 ug) are represented as the cationic/ionizable lipid component of each test article (*Formulations 1-4*)*.* Values are based on blood sample four hours post-administration.

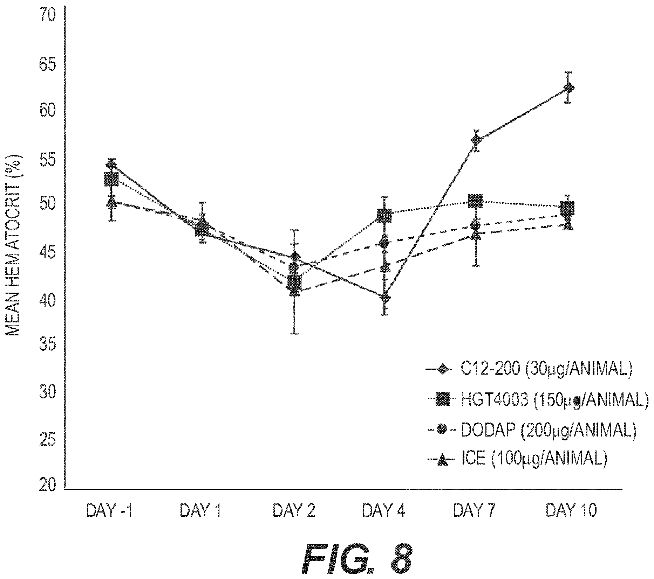

**FIG. 8** shows the hematocrit measurement of mice treated with a single IV dose of human EPO mRNA-loaded lipid nanoparticles (*Formulations 1-4*)*.* Whole blood samples were taken at 4 hr (Day 1), 24 hr (Day 2), 4 days, 7 days, and 10 days post-administration.

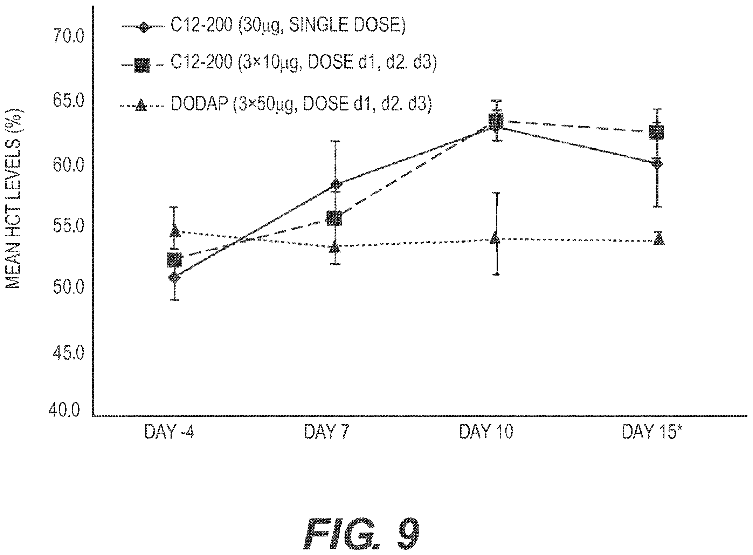

**FIG. 9** shows hematocrit measurements of mice treated with human EPO-mRNA-loaded lipid nanoparticles with either a single IV dose or three injections (day 1, day 3, day 5). Whole blood samples were taken prior to injection (day -4), day 7, and day 15. *Formulation 1* was administered: (30 ug, single dose) or (3 × 10 ug, dose day 1, day 3, day 5); *Formulation 2* was administered: (3 × 50 ug, dose day 1, day 3, day 5).

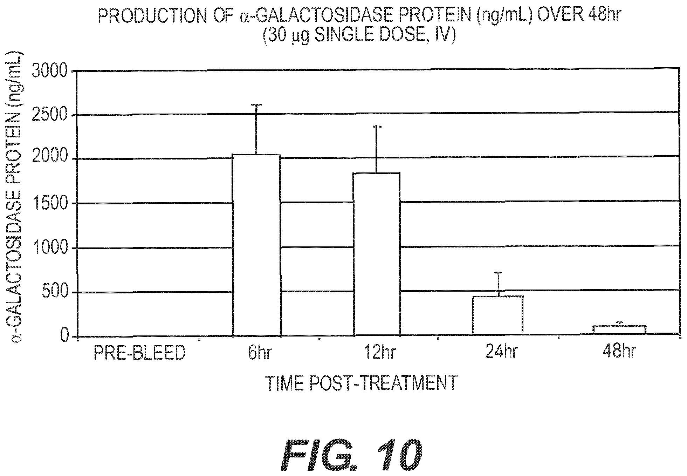

**FIG. 10** shows quantification of secreted human α-galactosidase (hGLA) protein levels as measured via ELISA. The protein detected is a result of the production from hGLA mRNA delivered via lipid nanoparticles *(Formulation 1*; 30 ug single intravenous dose, based on encapsulated mRNA). hGLA protein is detected through 48 hours.

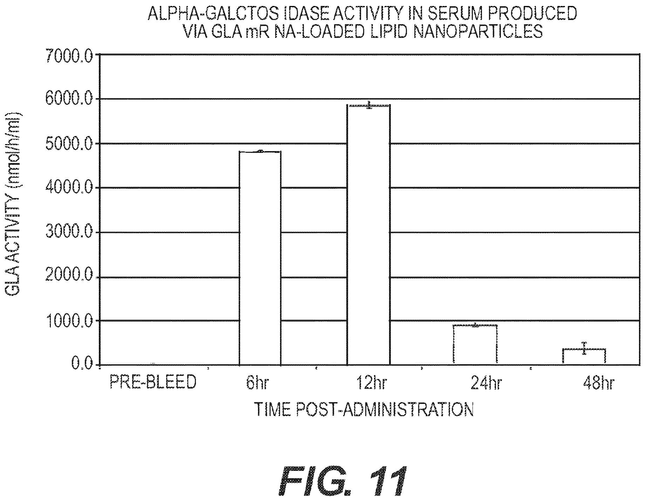

**FIG. 11** shows hGLA activity in serum. hGLA activity was measured using substrate 4-methylumbelliferyl-α-D-galactopyranoside (4-MU-α-gal) at 37°C. Data are average of 6 to 9 individual measurements.

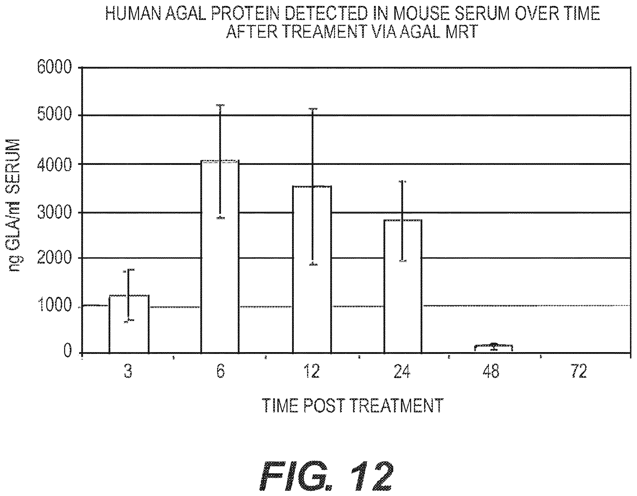

**FIG. 12** shows quantification of hGLA protein levels in serum as measured via ELISA. Protein is produced from hGLA mRNA delivered via C12-200-based lipid nanoparticles (C 12-200: DOPE: Chol: DMGPEG2K, 40:30:25:5 *(Formulation 1*); 30 ug mRNA based on encapsulated mRNA, single IV dose). hGLA protein is monitored through 72 hours.

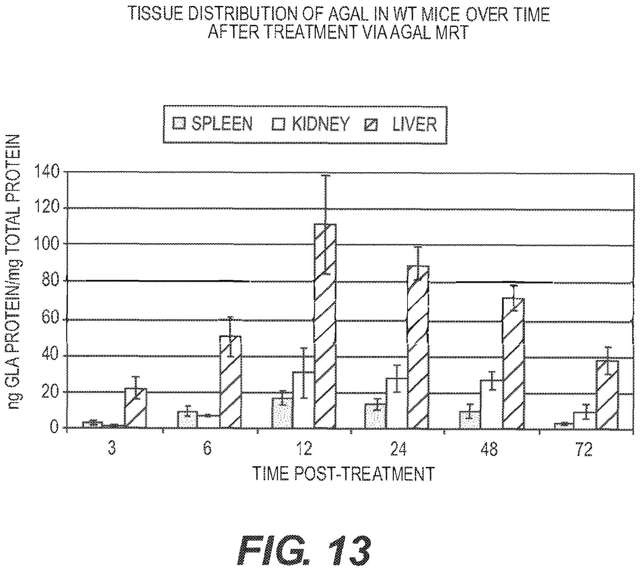

**FIG. 13** shows quantification of hGLA protein levels in liver, kidney, and spleen as measured via ELISA. Protein is produced from hGLA mRNA delivered via C12-200-based lipid nanoparticles *(Formulation* 1; 30 ug mRNA based on encapsulated mRNA, single IV dose). hGLA protein is monitored through 72 hours.

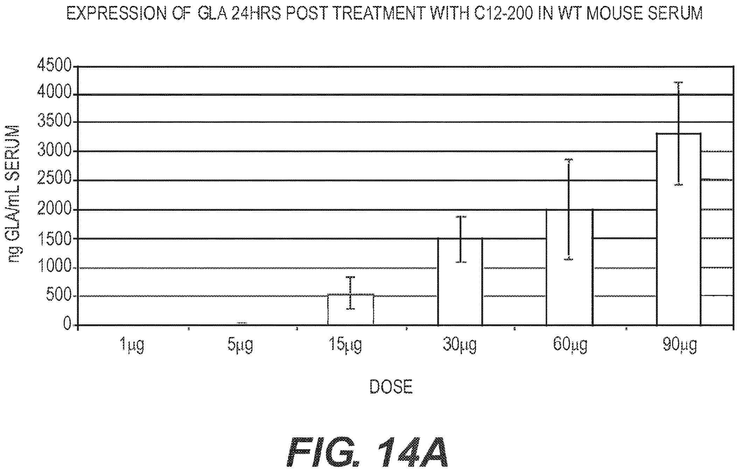

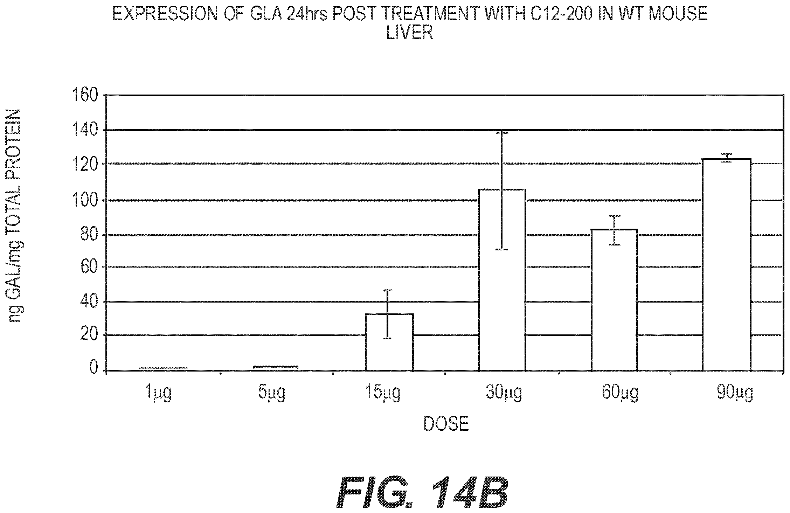

**FIG. 14** shows a dose response study monitoring protein production of hGLA as secreted MRT-derived human GLA protein in serum (A) and liver (B). Samples were measured 24 hours post-administration (*Formulation 1;* single dose, IV, N=4 mice/group) and quantified via ELISA.

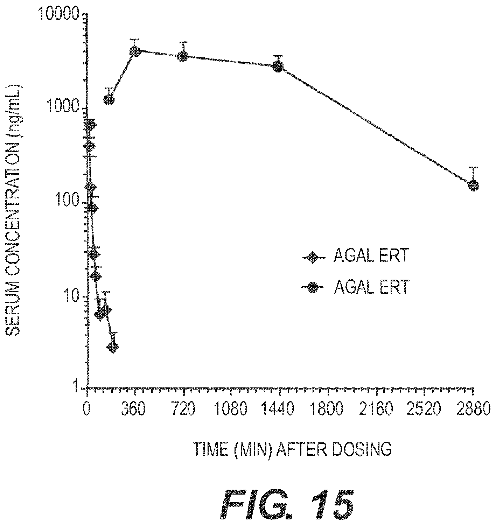

**FIG. 15** shows the pharmacokinetic profiles of ERT-based Alpha-galactosidase in athymic nude mice (40 ug/kg dose) and hGLA protein produced from MRT *(Formulation 1*; 1.0 mg/kg mRNA dose).

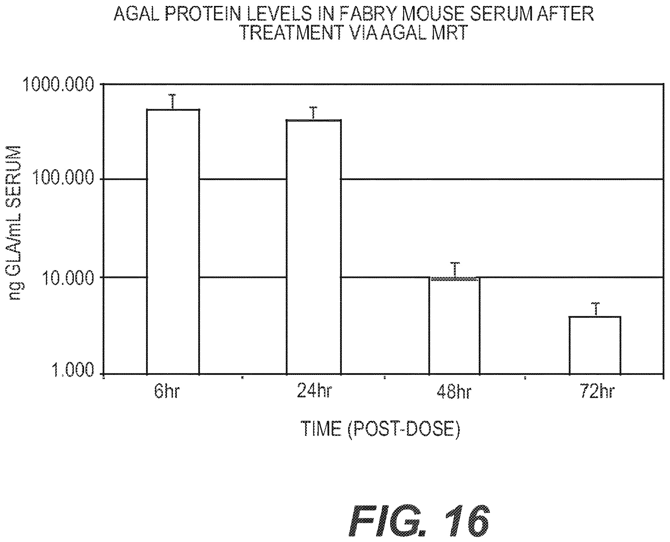

**FIG. 16** shows the quantification of secreted hGLA protein levels in MRT-treated Fabry mice as measured using ELISA. hGLA protein is produced from hGLA mRNA delivered via C12-200-based lipid nanoparticles *(Formulation 1*; 10 ug mRNA per single intravenous dose, based on encapsulated mRNA). Serum is monitored through 72 hours.

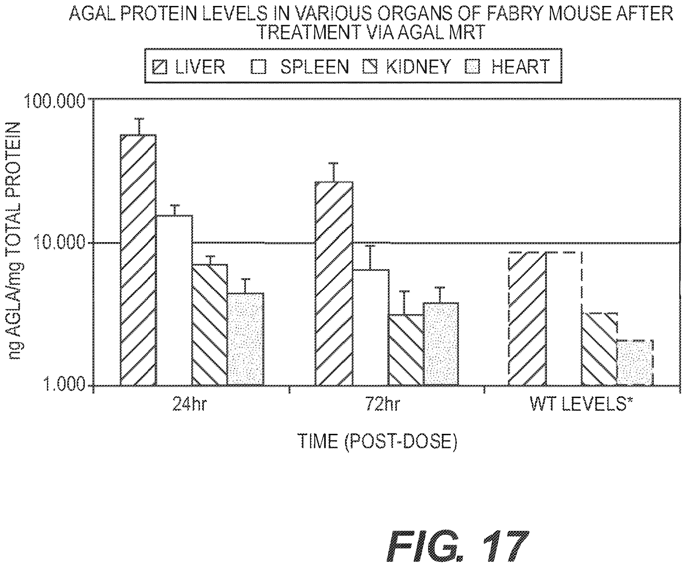

**FIG. 17** shows the quantification of hGLA protein levels in liver, kidney, spleen, and heart of MRT-treated Fabry KO mice as measured via ELISA. Protein is produced from hGLA mRNA delivered via C12-200-based lipid nanoparticles *(Formulation 1*; 30 ug mRNA based on encapsulated mRNA, single IV dose). hGLA protein is monitored through 72 hours. Literature values representing normal physiological levels are graphed as dashed lines.

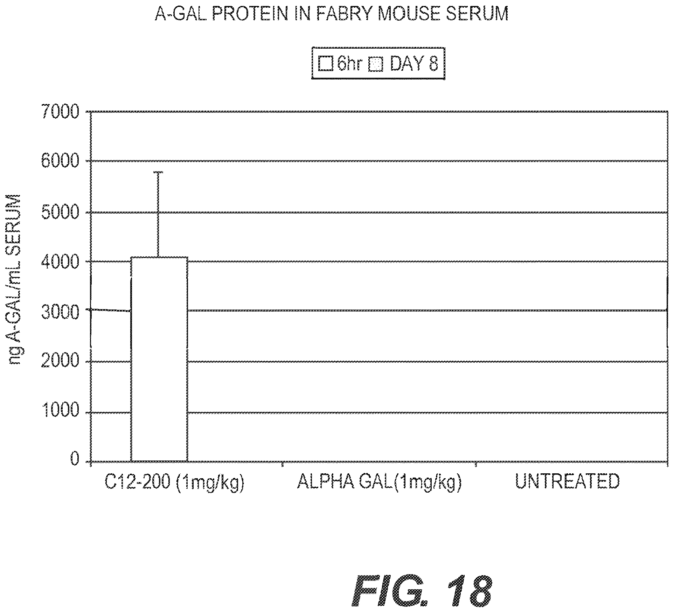

**FIG. 18** shows the quantification of secreted hGLA protein levels in MRT and Alpha-galactosidase-treated Fabry mice as measured using ELISA. Both therapies were dosed as a single 1.0 mg/kg intravenous dose.

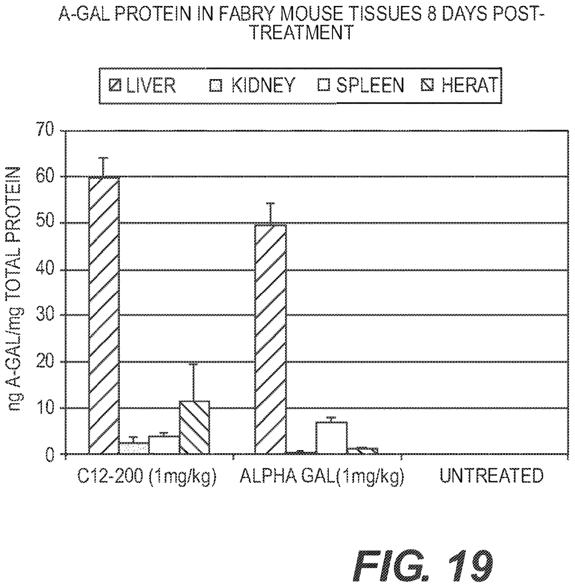

**FIG. 19** shows the quantification of hGLA protein levels in liver, kidney, spleen, and heart of MRT and ERT (Alpha-galactosidase)-treated Fabry KO mice as measured via ELISA. Protein produced from hGLA mRNA delivered via lipid nanoparticles *(Formulation 1*; 1.0 mg/kg mRNA based on encapsulated mRNA, single IV dose).

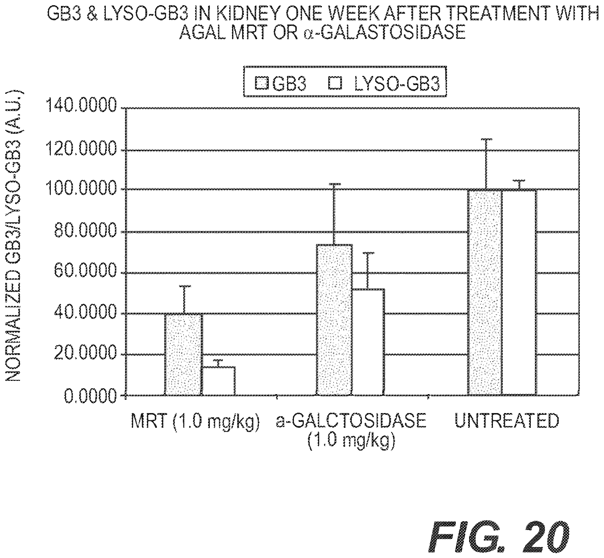

**FIG. 20** shows the relative quantification of globotrioasylceramide (Gb3) and lyso-Gb3 in the kidneys of treated and untreated mice. Male Fabry KO mice were treated with a single dose either GLA mRNA-loaded lipid nanoparticles or Alpha-galactosidase at 1.0 mg/kg. Amounts reflect quantity of Gb3/lyso-Gb3 one week post-administration.

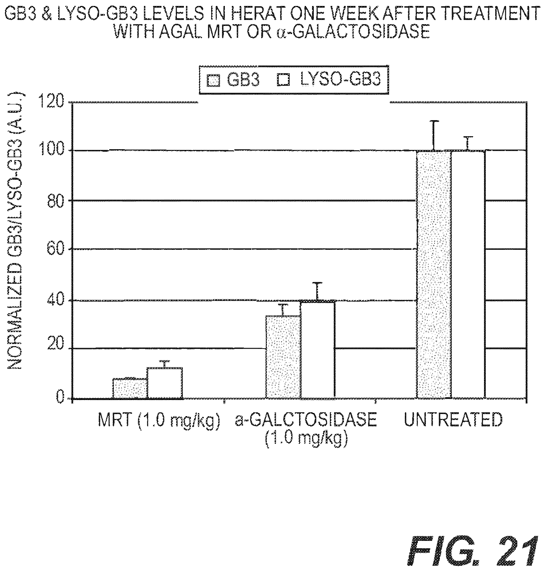

**FIG. 21** shows the relative quantification of globotrioasylceramide (Gb3) and lyso-Gb3 in the heart of treated and untreated mice. Male Fabry KO mice were treated with a single dose either GLA mRNA-loaded lipid nanoparticles or Alpha-galactosidase at 1.0 mg/kg. Amounts reflect quantity of Gb3/lyso-Gb3 one week post-administration.

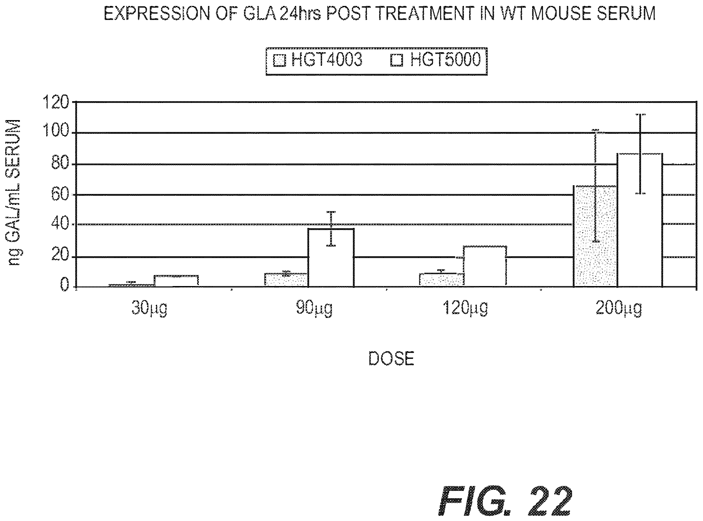

**FIG. 22** shows a dose response study monitoring protein production of GLA as secreted MRT-derived human GLA protein in serum. Samples were measured 24 hours post-administration (single dose, IV, N=4 mice/group) of either HGT4003 *(Formulation 3*) or HGT5000-based lipid nanoparticles (*Formulation 5*) and quantified via ELISA.

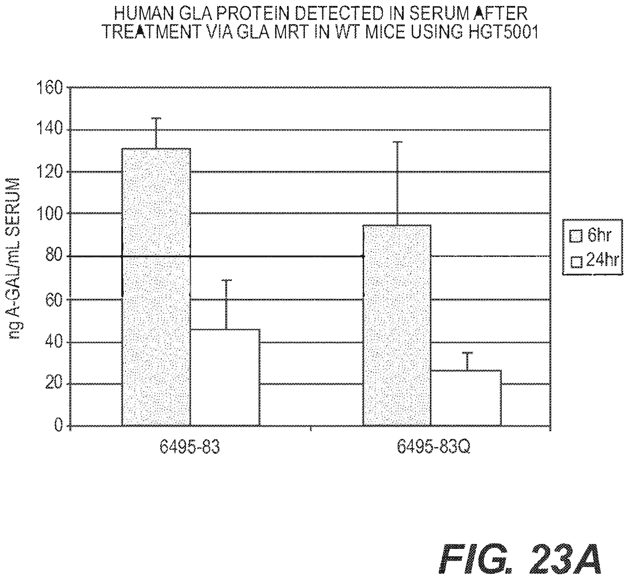

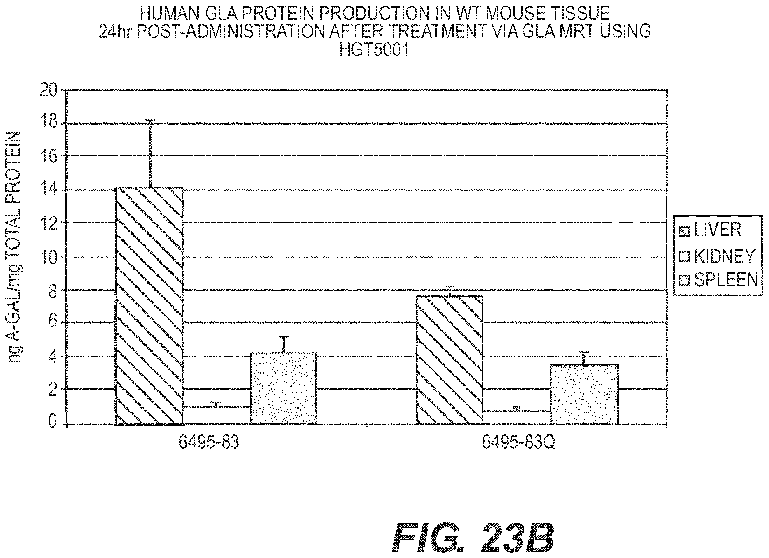

**FIG. 23** shows hGLA protein production as measured in serum (A) or in liver, kidney, and spleen (B). Samples were measured 6 hours and 24 hours post-administration (single dose, IV, N=4 mice/group) of HGT5001-based lipid nanoparticles *(Formulation 6*) and quantified via ELISA.

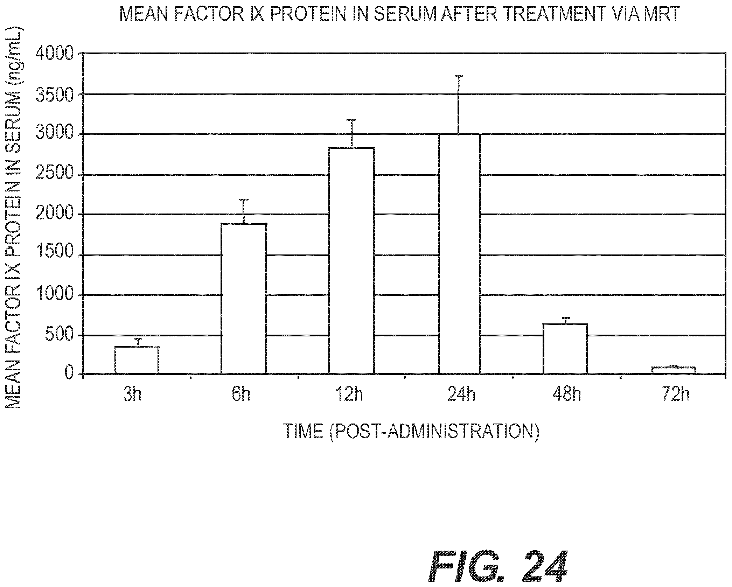

**FIG. 24** shows the quantification of secreted human Factor IX protein levels measured using ELISA (mean ng/mL ± standard deviation). FIX protein is produced from FIX mRNA delivered via C12-200-based lipid nanoparticles (C12-200:DOPE:Chol:DMGPEG2K, 40:30:25:5 *(Formulation 1*); 30 ug mRNA per single intravenous dose, based on encapsulated mRNA). FIX protein is monitored through 72 hours. (n=24 mice)

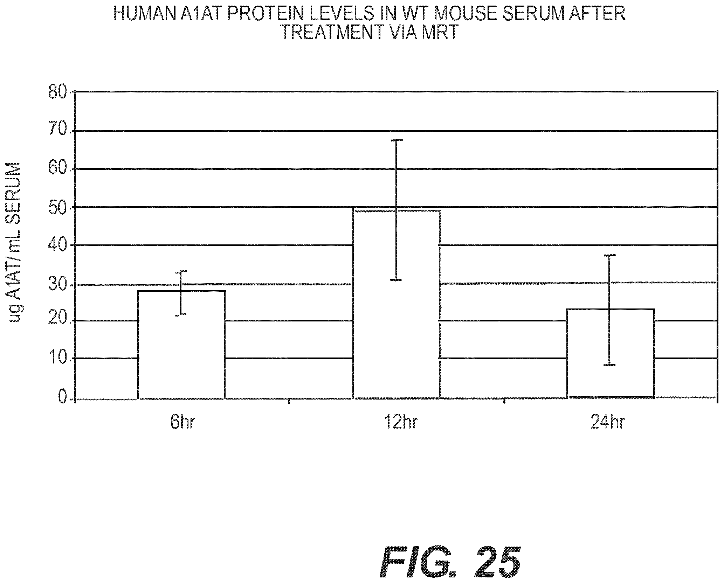

**FIG. 25** shows the quantification of secreted human α-1-antitrypsin (A1AT) protein levels measured using ELISA. A1AT protein is produced from A1AT mRNA delivered via C12-200-based lipid nanoparticles (C12-200:DOPE:Chol:DMGPEG2K, 40:30:25:5 *(Formulation 1*); 30 ug mRNA per single intravenous dose, based on encapsulated mRNA). A1AT protein is monitored through 24 hours.

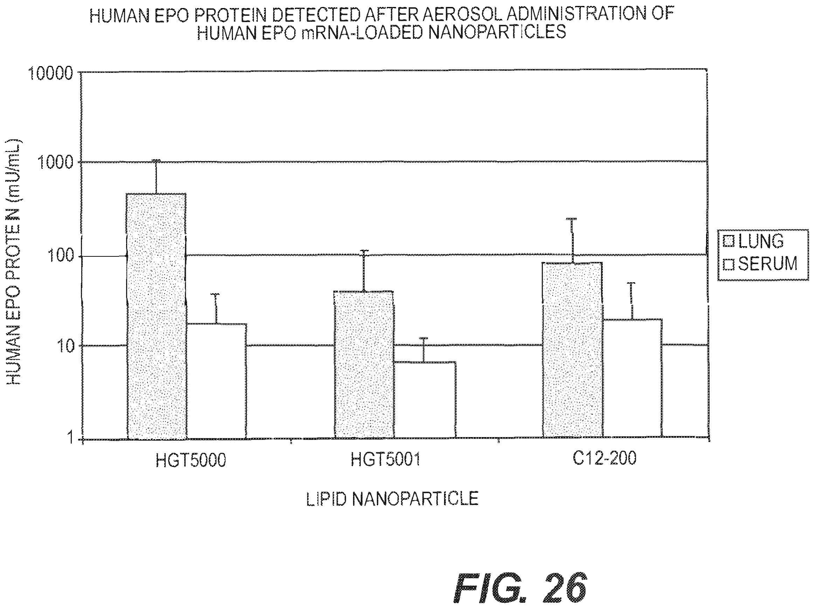

**FIG. 26** shows an ELISA-based quantification of hEPO protein detected in the lungs and serum of treated mice after intratracheal administration of hEPO mRNA-loaded nanoparticles (measured mill) (C12-200, HGT5000, or HGT5001- based lipid nanoparticles; *Formulations 1, 5, 6 respectively*)*.* Animals were sacrificed 6 hours post-administration (n=4 mice per group).

### DETAILED DESCRIPTION OF THE INVENTION

The invention defined in the appended claims provides a composition for use in a method of treating a disease in a subject that is due to a deficiency of a pulmonary surfactant-associated protein, wherein the pulmonary surfactant-associated protein is selected from SFTPA1, SFTPA2, SFTPB, SFTPC, and SFTPD, wherein said method comprises pulmonary administration of the composition to the subject, said composition comprising: (a) at least one mRNA molecule at least a portion of which encodes the pulmonary surfactant-associated protein; and (b) a transfer vehicle comprising a lipid nanoparticle, wherein the lipid nanoparticle comprises one or more cationic lipids, one or more non-cationic lipids and one of more PEG-modified lipids.

The term "functional," as used herein to qualify a protein, means that the protein has biological activity, or alternatively is able to perform the same, or a similar function as the native or normally-functioning protein. The term "therapeutic levels" refers to levels of the pulmonary surfactant-associated protein detected in the tissues that are above control levels, wherein the control may be normal physiological levels, or the levels in the subject prior to administration of the mRNA composition. The term "secreted" refers to the pulmonary surfactant-associated protein that is detected outside the target cell, in extracellular space. In the context of the present invention the term "produced" is used in its broadest sense to refer the translation of at least one mRNA into a pulmonary surfactant-associated protein. As provided herein, the compositions include a transfer vehicle. The transfer vehicles described herein are capable of delivering mRNA to the target cell. ***mRNA***

The mRNA in the compositions for the use according to the invention encode a pulmonary surfactant-associated protein. In some embodiments of the invention, the mRNA may optionally have chemical or biological modifications which, for example, improve the stability and/or half-life of such mRNA or which improve or otherwise facilitate protein production. The invention provides for optional co-delivery of more than one unique mRNA to target cells, for example, by combining two unique mRNAs into a single transfer vehicle. In one embodiment of the present invention, a therapeutic first mRNA, and a therapeutic second mRNA, may be formulated in a single transfer vehicle and administered. The present invention also contemplates co-delivery and/or co-administration of a therapeutic first mRNA and a second nucleic acid to facilitate and/or enhance the function or delivery of the therapeutic first mRNA. For example, the therapeutic first mRNA may be administered with a second nucleic acid that functions as a "chaperone" for example, to direct the folding of either the therapeutic first mRNA.

The invention also provides for the delivery of one or more therapeutic nucleic acids to treat a single disorder or deficiency, wherein each such therapeutic nucleic acid functions by a different mechanism of action. For example, the compositions for the use according to the present invention may comprise a therapeutic first mRNA which, for example, is administered to correct an endogenous pulmonary surfactant-associated protein deficiency, and which is accompanied by a second nucleic acid, which is administered to deactivate or "knock-down" a malfunctioning endogenous nucleic acid and its protein or enzyme product. Such "second" nucleic acids may encode, for example mRNA or siRNA.

Upon transfection, a natural mRNA in the compositions may decay with a half-life of between 30 minutes and several days. The mRNA preferably retain at least some ability to be translated, thereby producing a functional pulmonary surfactant-associated protein. Accordingly, it is envisioned to administer a stabilized mRNA. In some embodiments of the invention, the activity of the mRNA is prolonged over an extended period of time. For example, the activity of the mRNA may be prolonged such that the compositions are administered to a subject on a semi-weekly or bi-weekly basis, or more preferably on a monthly, bi-monthly, quarterly or an annual basis. The extended or prolonged activity of the mRNA is directly related to the quantity of pulmonary surfactant-associated protein produced from such mRNA. Similarly, the activity of the compositions may be further extended or prolonged by modifications made to improve or enhance translation of the mRNA. Furthermore, the quantity of functional protein produced by the target cell is a function of the quantity of mRNA delivered to the target cells and the stability of such mRNA. To the extent that the stability of the mRNA may be improved or enhanced, the half-life, the activity of the produced protein or enzyme and the dosing frequency of the composition may be further extended.

Accordingly, in some embodiments, the mRNA in the compositions for the use according to the invention comprise at least one modification which confers increased or enhanced stability to the nucleic acid, including, for example, improved resistance to nuclease digestion *in vivo.* As used herein, the terms "modification" and "modified" as such terms relate to the nucleic acids provided herein, include at least one alteration which preferably enhances stability and renders the mRNA more stable (e.g., resistant to nuclease digestion) than the wild-type or naturally occurring version of the mRNA. As used herein, the terms "stable" and "stability" as such terms relate to the nucleic acids of the present invention, and particularly with respect to the mRNA, refer to increased or enhanced resistance to degradation by, for example nucleases (i.e., endonucleases or exonucleases) which are normally capable of degrading such mRNA. Increased stability can include, for example, less sensitivity to hydrolysis or other destruction by endogenous enzymes (e.g., endonucleases or exonucleases) or conditions within the target cell or tissue, thereby increasing or enhancing the residence of such mRNA in the target cell, tissue, subject and/or cytoplasm. The stabilized mRNA molecules provided herein demonstrate longer half-lives relative to their naturally occurring, unmodified counterparts (e.g. the wild-type version of the mRNA). Also contemplated by the terms "modification" and "modified" as such terms related to the mRNA are alterations which improve or enhance translation of mRNA nucleic acids, including for example, the inclusion of sequences which function in the initiation of protein translation (e.g., the Kozak consensus sequence; see Kozak, M., Nucleic Acids Res 15 (20): 8125-48 (1987)).

In some embodiments, the mRNA has undergone a chemical or biological modification to render them more stable. Exemplary modifications to an mRNA include the depletion of a base (e.g., by deletion or by the substitution of one nucleotide for another) or modification of a base, for example, the chemical modification of a base. The phrase "chemical modifications" as used herein, includes modifications which introduce chemistries which differ from those seen in naturally occurring mRNA, for example, covalent modifications such as the introduction of modified nucleotides, (e.g., nucleotide analogs, or the inclusion of pendant groups which are not naturally found in such mRNA molecules).

In addition, suitable modifications include alterations in one or more nucleotides of a codon such that the codon encodes the same amino acid but is more stable than the codon found in the wild-type version of the mRNA. For example, an inverse relationship between the stability of RNA and a higher number cytidines (C's) and/or uridines (U's) residues has been demonstrated, and RNA devoid of C and U residues have been found to be stable to most RNases (Heidenreich, et al. J Biol Chem 269, 2131-8 (1994)). In some embodiments, the number of C and/or U residues in an mRNA sequence is reduced. In a another embodiment, the number of C and/or U residues is reduced by substitution of one codon encoding a particular amino acid for another codon encoding the same or a related amino acid. Contemplated modifications to the mRNA nucleic acids also include the incorporation of pseudouridines. The incorporation of pseudouridines into the mRNA nucleic acids may enhance stability and translational capacity, as well as diminishing immunogenicity *in vivo. See, e.g.,* Karikó, K., et al., Molecular Therapy 16 (11): 1833-1840 (2008). Substitutions and modifications to the mRNA may be performed by methods readily known to one or ordinary skill in the art.

The constraints on reducing the number of C and U residues in a sequence will likely be greater within the coding region of an mRNA, compared to an untranslated region, (i.e., it will likely not be possible to eliminate all of the C and U residues present in the message while still retaining the ability of the message to encode the desired amino acid sequence). The degeneracy of the genetic code, however presents an opportunity to allow the number of C and/or U residues that are present in the sequence to be reduced, while maintaining the same coding capacity (i.e., depending on which amino acid is encoded by a codon, several different possibilities for modification of RNA sequences may be possible). For example, the codons for Gly can be altered to GGA or GGG instead of GGU or GGC.

The term modification also includes, for example, the incorporation of non-nucleotide linkages or modified nucleotides into the mRNA sequences (e.g., modifications to one or both the 3' and 5' ends of an mRNA molecule encoding a functional pulmonary surfactant-associated protein). Such modifications include the addition of bases to an mRNA sequence (e.g., the inclusion of a poly A tail or a longer poly A tail), the alteration of the 3' UTR or the 5' UTR, complexing the mRNA with an agent (e.g., a protein or a complementary nucleic acid molecule), and inclusion of elements which change the structure of an mRNA molecule (e.g., which form secondary structures).

The poly A tail is thought to stabilize natural messengers. Therefore, in one embodiment a long poly A tail can be added to an mRNA molecule thus rendering the mRNA more stable. Poly A tails can be added using a variety of art-recognized techniques. For example, long poly A tails can be added to synthetic or *in vitro* transcribed mRNA using poly A polymerase (Yokoe, et al. Nature Biotechnology. 1996; 14: 1252-1256). A transcription vector can also encode long poly A tails. In addition, poly A tails can be added by transcription directly from PCR products. In one embodiment, the length of the poly A tail is at least about 90, 200, 300, 400 at least 500 nucleotides. In one embodiment, the length of the poly A tail is adjusted to control the stability of a modified mRNA molecule and, thus, the transcription of pulmonary surfactant-associated protein. For example, since the length of the poly A tail can influence the half-life of an mRNA molecule, the length of the poly A tail can be adjusted to modify the level of resistance of the mRNA to nucleases and thereby control the time course of protein expression in a cell.

In one embodiment, an mRNA can be modified by the incorporation 3' and/or 5' untranslated (UTR) sequences which are not naturally found in the wild-type mRNA. In one embodiment, 3' and/or 5' flanking sequence which naturally flanks an mRNA and encodes a second, unrelated protein can be incorporated into the nucleotide sequence of an mRNA molecule encoding a therapeutic or functional protein in order to modify it. For example, 3' or 5' sequences from mRNA molecules which are stable (e.g., globin, actin, GAPDH, tubulin, histone, or citric acid cycle enzymes) can be incorporated into the 3' and/or 5' region of a sense mRNA nucleic acid molecule to increase the stability of the sense mRNA molecule. *See, e.g.,* US2003/0083272.

In some embodiments, the mRNA in the compositions for the use according to the invention include modification of the 5' end of the mRNA to include a partial sequence of a CMV immediate-early 1 (IE1) gene, or a fragment thereof (e.g., SEQ ID NO: 1) to improve the nuclease resistance and/or improve the half-life of the mRNA. In addition to increasing the stability of the mRNA nucleic acid sequence, it has been surprisingly discovered the inclusion of a partial sequence of a CMV immediate-early 1 (IE1) gene enhances the translation of the mRNA and the expression of the functional protein or enzyme. Also contemplated is the inclusion of a human growth hormone (hGH) gene sequence, or a fragment thereof (e.g., SEQ ID NO:2) to the 3' ends of the nucleic acid (e.g., mRNA) to further stabilize the mRNA. Generally, preferred modifications improve the stability and/or pharmacokinetic properties (e.g., half-life) of the mRNA relative to their unmodified counterparts, and include, for example modifications made to improve such mRNA's resistance to *in vivo* nuclease digestion.

Further contemplated are variants of the nucleic acid sequence of SEQ ID NO: 1 and/or SEQ ID NO:2, wherein the variants maintain the functional properties of the nucleic acids including stabilization of the mRNA and/or pharmacokinetic properties (e.g., half-life). Variants may have greater than 90%, greater than 95%, greater than 98%, or greater than 99% sequence identity to SEQ ID NO: 1 or SEQ ID NO:2.

When RNA is hybridized to a complementary nucleic acid molecule (e.g., DNA or RNA) it may be protected from nucleases. (Krieg, et al. Melton. Methods in Enzymology. 1987; 155, 397-415). The stability of hybridized mRNA is likely due to the inherent single strand specificity of most RNases. In some embodiments, the stabilizing reagent selected to complex a mRNA is a eukaryotic protein, (e.g., a mammalian protein). In yet another embodiment, the mRNA can be modified by hybridization to a second nucleic acid molecule. If an entire mRNA molecule were hybridized to a complementary nucleic acid molecule translation initiation may be reduced. In some embodiments the 5' untranslated region and the AUG start region of the mRNA molecule may optionally be left unhybridized. Following translation initiation, the unwinding activity of the ribosome complex can function even on high affinity duplexes so that translation can proceed. (Liebhaber. J. Mol. Biol. 1992; 226: 2-13; Monia, et al. J Biol Chem. 1993; 268: 14514-22.)

It will be understood that any of the above described methods for enhancing the stability of mRNA may be used either alone or in combination with one or more of any of the other above-described methods and/or compositions.

The mRNA to be included in the composition for the use according to the invention may be optionally combined with a reporter gene (e.g., upstream or downstream of the coding region of the mRNA) which, for example, facilitates the determination of mRNA delivery to the target cells or tissues. Suitable reporter genes may include, for example, Green Fluorescent Protein mRNA (GFP mRNA), *Renilla* Luciferase mRNA (Luciferase mRNA), Firefly Luciferase mRNA, or any combinations thereof. For example, GFP mRNA may be fused with a mRNA encoding a secretable protein to facilitate confirmation of mRNA localization in the target cells that will act as a depot for protein production.

As used herein, the terms "transfect" or "transfection" mean the intracellular introduction of a mRNA into a cell, or preferably into a target cell. The introduced mRNA may be stably or transiently maintained in the target cell. The term "transfection efficiency" refers to the relative amount of mRNA taken up by the target cell which is subject to transfection. In practice, transfection efficiency is estimated by the amount of a reporter nucleic acid product expressed by the target cells following transfection. Preferred embodiments include compositions with high transfection efficacies and in particular those compositions that minimize adverse effects which are mediated by transfection of non-target cells. The compositions that demonstrate high transfection efficacies improve the likelihood that appropriate dosages of the mRNA are particularly suitable for delivery to the target cell, while minimizing potential systemic adverse effects. In one embodiment of the present invention, the transfer vehicles are capable of delivering large mRNA sequences (e.g., mRNA of at least 1kDa, 1.5kDa, 2 kDa, 2.5kDa, 5kDa, 10kDa, 12kDa, 15kDa, 20kDa, 25kDa, 30kDa, or more, or alternatively mRNA of a size ranging from 0.2 kilobases (kb) to 10 kb or more, e.g., mRNA of a size greater than or equal to 0.2 kb, 0.5 kb, 1 kb, 1.5 kb, 2 kb, 3 kb, 4 kb, or 4.5 kb, and/or having a size of up to 5 kb, 5.5 kb, 6 kb, 7 kb, 8 kb, 9 kb, or 10 kb). The mRNA can be formulated with one or more acceptable reagents, which provide a vehicle for delivering such mRNA to target cells. Appropriate reagents are generally selected with regard to a number of factors, which include, among other things, the biological or chemical properties of the mRNA, the intended route of administration, the anticipated biological environment to which such mRNA will be exposed and the specific properties of the intended target cells. In some embodiments, the transfer vehicle demonstrates preferential and/or substantial binding to a target cell relative to non-target cells. In a preferred embodiment, the transfer vehicle delivers its contents to the target cell such that the mRNA are delivered to the appropriate subcellular compartment, such as the cytoplasm.

### Transfer Vehicle

The transfer vehicle in the compositions of the invention is a liposomal transfer vehicle, e.g. a lipid nanoparticle or a lipidoid nanoparticle. In one embodiment, the transfer vehicle may be selected and/or prepared to optimize delivery of the mRNA to a target cell.

Liposomes (e.g., liposomal lipid nanoparticles) are generally useful in a variety of applications in research, industry, and medicine, particularly for their use as transfer vehicles of diagnostic or therapeutic compounds *in vivo* (Lasic, Trends Biotechnol., 16: 307-321, 1998; Drummond et al., Pharmacol. Rev., 51: 691-743, 1999) and are usually characterized as microscopic vesicles having an interior aqua space sequestered from an outer medium by a membrane of one or more bilayers. Bilayer membranes of liposomes are typically formed by amphiphilic molecules, such as lipids of synthetic or natural origin that comprise spatially separated hydrophilic and hydrophobic domains (Lasic, Trends Biotechnol., 16: 307-321, 1998).

In the context of the present invention, a liposomal transfer vehicle typically serves to transport the mRNA to the target cell. For the purposes of the present invention, the liposomal transfer vehicles are prepared to contain the mRNA. The process of incorporation of an mRNA into a liposome is often referred to as "loading" (Lasic, et al., FEBS Lett., 312: 255-258, 1992). The liposome-incorporated mRNA may be completely or partially located in the interior space of the liposome, within the bilayer membrane of the liposome, or associated with the exterior surface of the liposome membrane. The incorporation of a nucleic acid into liposomes is also referred to herein as "encapsulation" wherein the nucleic acid is entirely contained within the interior space of the liposome. The purpose of incorporating a mRNA into a transfer vehicle, such as a liposome, is often to protect the nucleic acid from an environment which may contain enzymes or chemicals that degrade nucleic acids and/or systems or receptors that cause the rapid excretion of the nucleic acids. Accordingly, in a preferred embodiment of the present invention, the selected transfer vehicle is capable of enhancing the stability of the mRNA contained therein. The liposome can allow the encapsulated mRNA to reach the target cell and/or may preferentially allow the encapsulated mRNA to reach the target cell, or alternatively limit the delivery of such mRNA to other sites or cells where the presence of the administered mRNA may be useless or undesirable. Furthermore, incorporating the mRNA into a transfer vehicle also facilitates the delivery of such mRNA into a target cell.

Ideally, liposomal transfer vehicles are prepared to encapsulate one or more desired mRNA such that the compositions demonstrate a high transfection efficiency and enhanced stability.

### Lipid Nanoparticles

In the present invention, the transfer vehicle is formulated as a lipid nanoparticle. As used herein, the phrase "lipid nanoparticle" refers to a transfer vehicle comprising one or more lipids (e.g., cationic lipids, non-cationic lipids, and PEG-modified lipids). The lipid nanoparticle is formulated to deliver one or more mRNA to one or more target cells. Examples of suitable lipids include, for example, the phosphatidyl compounds (e.g., phosphatidylglycerol, phosphatidylcholine, phosphatidylserine, phosphatidylethanolamine, sphingolipids, cerebrosides, and gangliosides). The transfer vehicle is selected based upon its ability to facilitate the transfection of a mRNA to a target cell.

In the present invention, the composition uses lipid nanoparticles as transfer vehicles comprising a cationic lipid to encapsulate and/or enhance the delivery of mRNA into the target cell that will act as a depot for protein production. As used herein, the phrase "cationic lipid" refers to any of a number of lipid species that carry a net positive charge at a selected pH, such as physiological pH. The lipid nanoparticles are prepared by including multi-component lipid mixtures of varying ratios employing one or more cationic lipids, non-cationic lipids and PEG-modified lipids. Several cationic lipids have been described in the literature, many of which are commercially available.

Particularly suitable cationic lipids for use in carrying out the invention include those described in international patent publication WO 2010/053572, and most particularly, C12-200 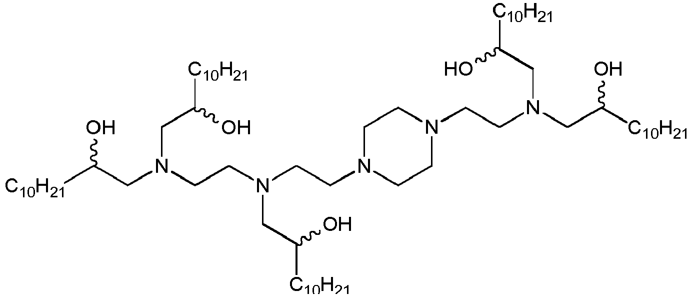 which is described at paragraph [00225] of WO 2010/053572.

In certain embodiments, the compositions employ a lipid nanoparticles comprising an ionizable cationic lipid described in U.S. provisional patent application 61/617,468, filed March 29, 2012 , such as, e.g., (15Z, 18Z)-N,N-dimethyl-6-(9Z, 12Z)-octadeca-9, 12-dien-1-yl)tetracosa-15, 18-dien-1-amine (HGT5000), (15Z, 18Z)-N,N-dimethyl-6-((9Z, 12Z)-octadeca-9, 12-dien-1-yl)tetracosa-4,15,18-trien-1-amine (HGT5001), and (15Z,18Z)-N,N-dimethyl-6-((9Z, 12Z)-octadeca-9, 12-dien-1-yl)tetracosa-5, 15, 18-trien-1-amine (HGT5002).

In some embodiments, the cationic lipid(s) is biodegradable and is a compound of formula (I): 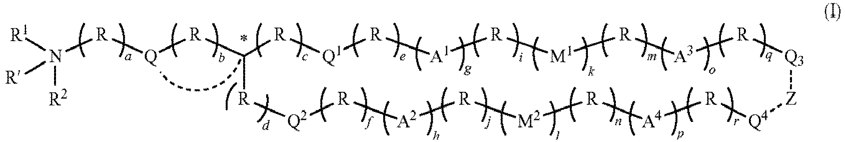 or a salt thereof,
wherein
R' is absent, hydrogen, or alkyl (e.g., C1-C4 alkyl);
with respect to R1 and R2,
   (i) R1 and R2 are each, independently, optionally substituted alkyl, alkenyl, alkynyl, cycloalkyl, cycloalkylalkyl, or heterocycle;
   (ii) R1 and R2, together with the nitrogen atom to which they are attached, form an optionally substituted heterocylic ring; or
   (iii) one of R1 and R2 is optionally substituted alkyl, alkenyl, alkynyl, cycloalkyl, cycloalkylalkyl, or heterocycle, and the other forms a 4-10 member heterocyclic ring or heteroaryl with (a) the adjacent nitrogen atom and (b) the (R)*ₐ* group adjacent to the nitrogen atom;
each occurrence of R is, independently, _(CR3R4)_;
each occurrence of R3and R4are, independently H, OH, alkyl, alkoxy, -NH2, alkylamino, or dialkylamino;
or R3 and R4, together with the carbon atom to which they are directly attached, form a cycloalkyl group, wherein
no more than three R groups in each chain attached to the carbon C* are cycloalkyl (e.g., cyclopropyl);
the dashed line to Q is absent or a bond;
when the dashed line to Q is absent, then Q is absent or is -0-, -S-, -C(O)O-, -OC(O)-, -C(O)N(R4)-, -N(R5)C(0)-, -S-S-, -OC(O)O-, -O-N=C(R₅)-, -C(R5) N-O-, -OC(O)N(R5)-, -N(R5)C(0)N(R5), -N(R5)C(0)0-, -C(O)S-, -C(S)O- or -C(R5) N-O-C(O)-;
   or
when the dashed line to Q is a bond, then b is °and Q and the tertiary carbon adjacent to it (C*) form a substituted or unsubstituted, mono- or bi-cyclic heterocyclic group having from 5 to 10 ring atoms;
Ql and Q2 are each, independently, absent, -0-, -S-, -OC(O)-, -C(O)O-, -SC(O)-, - C(O)S-, -OC(S)-, -C(S)O-, -S-S-, -C(0)(NR5)-, -N(R5)C(0)-, -C(S)(NR5)-, -N(R5)C(0)-, -N(R5)C(0)N(R5)-, or -OC(O)O-;
Q3 and Q4 are each, independently, H, -(CR3R4)-, aryl, or a cholesterol moiety;
each occurrence of A1, A2, A3 and A4 is, independently, -(CR5R5-CR5=CR5)-;
each occurrence of R5 is, independently, H or alkyl;
M1 and M2 are each, independently, a biodegradable group;
Z is absent, alkylene or -O-P(O)(OH)-O-;
each ------ attached to Z is an optional bond, such that when Z is absent, Q3 and Q4 are not directly covalently bound together;
a is 1,2,3,4,5 or 6;
b is 0, 1,2, or 3;
c, d, e, f, i, j, m, n, q and r are each, independently, 0, 1, 2, 3,4,5,6,7,8,9, or 10;
g and h are each, independently, 0, 1 or 2;
k and I are each, independently, °or I, where at least one of
k and I is I; and
o and p are each, independently, 0, 1 or 2.

Specific biodegradable lipids suitable for use in the compositions for the purposes of the invention include 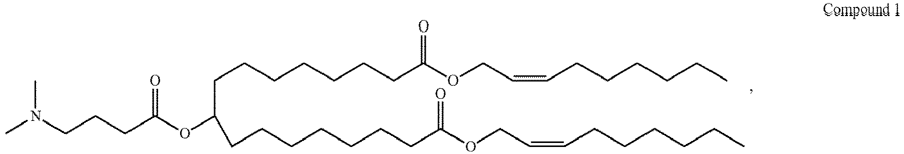 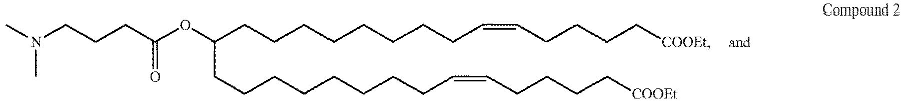 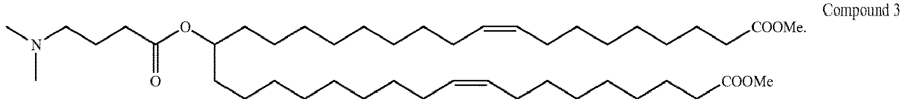 and their salts. Other specific biodegradable cationic lipids falling within formula I, such as compounds of any of formula I-XXIII, including compounds of formula IA-1, IA-2, IB, IC, or ID, as described in US 2012/0027803.

Other cationic lipids suitable for the purposes of the invention are described in US 20100267806. For example, lipids of formula II: 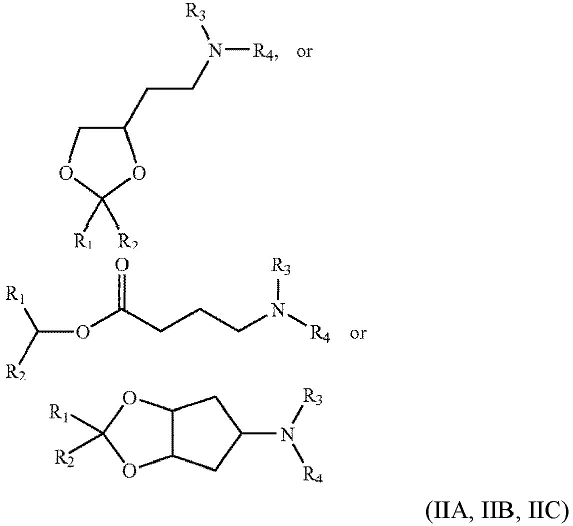 where R1 and R2 are independently alkyl, alkenyl or alkynyl, each can be optionally substituted, and R3 and R4 are independently lower alkyl or R3 and R4 can be taken together to form an optionally substituted heterocyclic ring. Specific cationic lipids suitable for the purposes of the invention are XTC (2,2-Dilinoley1-4-dimethylaminoethy1-[1,3]-dioxolane) and, MC3 (((6Z,9Z,28Z,3IZ)-heptatriaconta-6,9,28,31-tetraen-19-yl 4-(dimethylamino) butanoate): 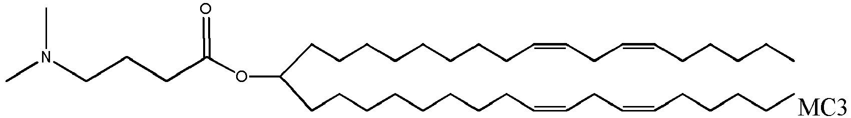 both of which are described in detail in US 20100267806. Another cationic lipid that may be used for the purposes of the invention is NC98-5 (4,7,13-tris(3-oxo-3-(undecylamino)propyl)-N1,N16-diundecyl-4,7,10, 13-tetraazahexadecane-1,16-diamide): 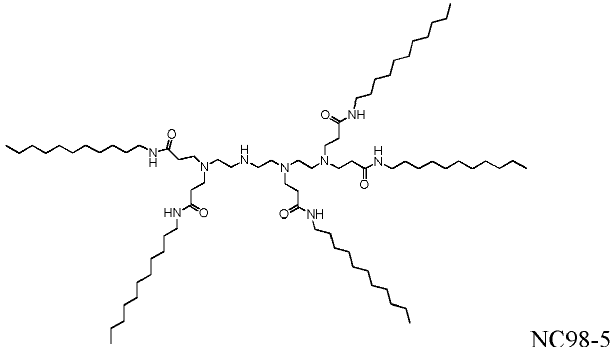 which is described in WO06138380A2.

In some embodiments, the cationic lipid N-[1-(2,3-dioleyloxy)propyl]-N,N,N-trimethylammonium chloride or "DOTMA" is used. (Felgner *et al.* (Proc. Nat'l Acad. Sci. 84, 7413 (1987); U.S. Pat. No. 4,897,355). DOTMA can be formulated alone or can be combined with the neutral lipid, dioleoylphosphatidylethanolamine or "DOPE" or other cationic or non-cationic lipids into a liposomal transfer vehicle (a lipid nanoparticle in the context of the invention), and such liposomes can be used to enhance the delivery of nucleic acids into target cells. Other suitable cationic lipids include, for example, 5-carboxyspermylglycinedioctadecylamide or "DOGS," 2,3-dioleyloxy-N-[2(spermine-carboxamido)ethyl]-N,N-dimethyl-1-propanaminium or "DOSPA" (Behr et al. Proc. Nat'l Acad. Sci. 86, 6982 (1989); U.S. Pat. No. 5,171,678; U.S. Pat. No. 5,334,761), 1,2-Dioleoyl-3-Dimethylammonium-Propane or "DODAP", 1,2-Dioleoyl-3-Trimethylammonium-Propane or "DOTAP". Contemplated cationic lipids also include 1,2-distearyloxy-N,N-dimethyl-3-aminopropane or "DSDMA", 1,2-dioleyloxy-N,N-dimethyl-3-aminopropane or "DODMA", 1,2-dilinoleyloxy-N,N-dimethyl-3-aminopropane or "DLinDMA", 1,2-dilinolenyloxy-N,N-dimethyl-3-aminopropane or "DLenDMA", N-dioleyl-N,N-dimethylammonium chloride or "DODAC", N,N-distearyl-N,N-dimethylammonium bromide or "DDAB", N-(1,2-dimyristyloxyprop-3-yl)-N,N-dimethyl-N-hydroxyethyl ammonium bromide or "DMRIE", 3-dimethylamino-2-(cholest-5-en-3-beta-oxybutan-4-oxy)-1-(cis,cis-9,12-octadecadienoxy)propane or "CLinDMA", 2-[5'-(cholest-5-en-3-beta-oxy)-3'-oxapentoxy)-3-dimethy l-1-(cis,cis-9', 1-2'-octadecadienoxy)propane or "CpLinDMA", N,N-dimethyl-3,4-dioleyloxybenzylamine or "DMOBA", 1,2-N,N'-dioleylcarbamyl-3-dimethylaminopropane or "DOcarbDAP", 2,3-Dilinoleoyloxy-N,N-dimethylpropylamine or "DLinDAP", 1,2-N,N'-Dilinoleylcarbamyl-3-dimethylaminopropane or "DLincarbDAP", 1,2-Dilinoleoylcarbamyl-3-dimethylaminopropane or "DLinCDAP", 2,2-dilinoleyl-4-dimethylaminomethyl-[1,3]-dioxolane or "DLin-K-DMA", 2,2-dilinoleyl-4-dimethylaminoethyl-[1,3]-dioxolane or "DLin-K-XTC2-DMA", and 2-(2,2-di((9Z,12Z)-octadeca-9,12-dien-1-yl)-1,3-dioxolan-4-yl)-N,N-dimethylethanamine (DLin-KC2-DMA)) (See, WO 2010/042877; Semple et al., Nature Biotech. 28:172-176 (2010)), or mixtures thereof. (Heyes, J., et al., J Controlled Release 107: 276-287 (2005); Morrissey, DV., et al., Nat. Biotechnol. 23(8): 1003-1007 (2005); PCT Publication WO2005/121348A1).

Cholesterol-based cationic lipids are also suitable for the purpose of carrying out the present invention. Such cholesterol-based cationic lipids can be used, either alone or in combination with other cationic or non-cationic lipids. Suitable cholesterol-based cationic lipids include, for example, DC-Chol (N,N-dimethyl-N-ethylcarboxamidocholesterol), 1,4-bis(3-N-oleylamino-propyl)piperazine (Gao, et al. Biochem. Biophys. Res. Comm. 179, 280 (1991); Wolf et al. BioTechniques 23, 139 (1997); U.S. Pat. No. 5,744,335), or ICE.

Also contemplated are cationic lipids such as the dialkylamino-based, imidazole-based, and guanidinium-based lipids. For example, certain embodiments are directed to a composition comprising one or more imidazole-based cationic lipids, for example, the imidazole cholesterol ester or "ICE" lipid (3S, 10R, 13R, 17R)-10, 13-dimethyl-17-((R)-6-methylheptan-2-yl)-2, 3, 4, 7, 8, 9, 10, 11, 12, 13, 14, 15, 16, 17-tetradecahydro-1H-cyclopenta[a]phenanthren-3-yl 3-(1H-imidazol-4-yl)propanoate, as represented by structure (I) below. In a preferred embodiment, a transfer vehicle for delivery of mRNA may comprise one or more imidazole-based cationic lipids, for example, the imidazole cholesterol ester or "ICE" lipid (3S, 10R, 13R, 17R)-10, 13-dimethyl-17-((R)-6-methylheptan-2-yl)-2, 3, 4, 7, 8, 9, 10, 11, 12, 13, 14, 15, 16, 17-tetradecahydro-1H-cyclopenta[a]phenanthren-3-yl 3-(1H-imidazol-4-yl)propanoate:

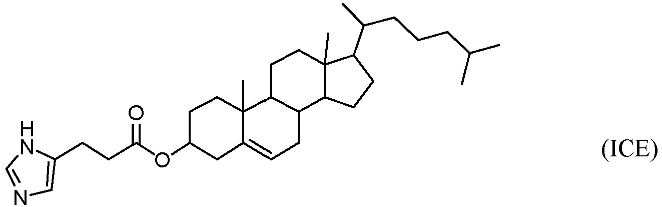

Without wishing to be bound by a particular theory, it is believed that the fusogenicity of the imidazole-based cationic lipid ICE is related to the endosomal disruption which is facilitated by the imidazole group, which has a lower pKa relative to traditional cationic lipids. The endosomal disruption in turn promotes osmotic swelling and the disruption of the liposomal membrane, followed by the transfection or intracellular release of the nucleic acid(s) contents loaded therein into the target cell.

The imidazole-based cationic lipids are also characterized by their reduced toxicity relative to other cationic lipids. The imidazole-based cationic lipids (e.g., ICE) may be used as the sole cationic lipid in the lipid nanoparticle, or alternatively may be combined with traditional cationic lipids, non-cationic lipids, and PEG-modified lipids. The cationic lipid(s) may comprise a molar ratio of about 1% to about 90%, about 2% to about 70%, about 5% to about 50%, about 10% to about 40% of the total lipid present in the transfer vehicle, or preferably about 20% to about 70% of the total lipid present in the transfer vehicle.

Similarly, certain embodiments are directed to lipid nanoparticles comprising the HGT4003 cationic lipid 2-((2,3-Bis((9Z, 12Z)-octadeca-9,12-dien-1-yloxy)propyl)disulfanyl)-N,N-dimethylethanamine, as represented by structure (IV) below, and as further described in U.S. Provisional Application No:61/494,745, filed June 8, 2011:

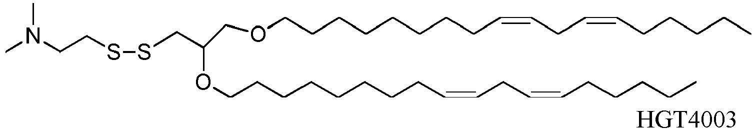

In other embodiments the compositions and methods described herein are directed to lipid nanoparticles comprising one or more cleavable lipids, such as, for example, one or more cationic lipids or compounds that comprise a cleavable disulfide (S-S) functional group (e.g., HGT4001, HGT4002, HGT4003, HGT4004 and HGT4005), as further described in U.S. Provisional Application No: 61/494,745.

Polyethylene glycol (PEG)-modified phospholipids and derivatized lipids such as derivatized ceramides (PEG-CER), including N-Octanoyl-Sphingosine-1-[Succinyl(Methoxy Polyethylene Glycol)-2000] (C8 PEG-2000 ceramide) can also be used for the purposes of the present invention, either alone or preferably in combination with other lipids together which comprise the transfer vehicle (e.g., a lipid nanoparticle). Contemplated PEG-modified lipids include, but is not limited to, a polyethylene glycol chain of up to 5 kDa in length covalently attached to a lipid with alkyl chain(s) of C₆-C₂₀ length. The addition of such components may prevent complex aggregation and may also provide a means for increasing circulation lifetime and increasing the delivery of the lipid-nucleic acid composition to the target cell, (Klibanov et al. (1990) FEBS Letters, 268 (1): 235-237), or they may be selected to rapidly exchange out of the formulation *in vivo* (see U.S. Pat. No. 5,885,613). Particularly useful exchangeable lipids are PEG-ceramides having shorter acyl chains (e.g., C14 or C18). The PEG-modified phospholipid(s) and derivatized lipids may comprise a molar ratio from about 0% to about 20%, about 0.5% to about 20%, about 1% to about 15%, about 4% to about 10%, or about 2% of the total lipid present in the liposomal transfer vehicle.

The present invention also contemplates the use of non-cationic lipids. As used herein, the phrase "non-cationic lipid" refers to any neutral, zwitterionic or anionic lipid. As used herein, the phrase "anionic lipid" refers to any of a number of lipid species that carry a net negative charge at a selected pH, such as physiological pH. Non-cationic lipids include, but are not limited to, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoylphosphatidylethanolamine (DOPE), palmitoyloleoylphosphatidylcholine (POPC), palmitoyloleoyl-phosphatidylethanolamine (POPE), dioleoyl-phosphatidylethanolamine 4-(N-maleimidomethyl)-cyclohexane-1-carboxylate (DOPE-mal), dipalmitoyl phosphatidyl ethanolamine (DPPE), dimyristoylphosphoethanolamine (DMPE), distearoyl-phosphatidyl-ethanolamine (DSPE), 16-0-monomethyl PE, 16-O-dimethyl PE, 18-1-trans PE, 1-stearoyl-2-oleoyl-phosphatidyethanolamine (SOPE), cholesterol, or a mixture thereof. The non-cationic lipid may comprise a molar ratio of 5% to about 90%, or preferably about 10 % to about 70% of the total lipid present in the transfer vehicle.

Preferably, the transfer vehicle (i.e., a lipid nanoparticle) is prepared by combining multiple lipid components. For example, a transfer vehicle may comprise OTC, DSPC, chol, and DMG-PEG or MC3, DSPC, chol, and DMG-PEG or C12-200, DOPE, chol, DMG-PEG2K. The selection of cationic lipids, non-cationic lipids and PEG-modified lipids which comprise the lipid nanoparticle, as well as the relative molar ratio of such lipids to each other, is based upon the characteristics of the selected lipids, the nature of the intended target cells, the characteristics of the mRNA to be delivered. For example, a transfer vehicle may be prepared using C12-200, DOPE, chol, DMG-PEG2K at a molar ratio of 40:30:25:5; or DODAP, DOPE, cholesterol, DMG-PEG2K at a molar ratio of 18:56:20:6; or HGT5000, DOPE, chol, DMG-PEG2K at a molar ratio of 40:20:35:5; or HGT5001, DOPE, chol, DMG-PEG2K at a molar ratio of 40:20:35:5; or XTC, DSPC, chol, PEG-DMG at a molar ratio of 57.5:7.5:31.5:3.5 or a molar ratio of 60:7.5:31:1.5; or MC3, DSPC, chol, PEG-DMG in a molar ratio of 50:10:38.5:1.5 or a molar ratio of 40:15:40:5; or MC3, DSPC, chol, PEG-DSG/GalNAc-PEGDSG in a molar ratio of 50:10:35:4.5:0.5.

Additional considerations include, for example, the saturation of the alkyl chain, as well as the size, charge, pH, pKa, fusogenicity and toxicity of the selected lipid(s). Thus the molar ratios may be adjusted accordingly. For example, the percentage of cationic lipid(s) in the lipid nanoparticle may be greater than 10%, greater than 20%, greater than 30%, greater than 40%, greater than 50%, greater than 60%, or greater than 70%. The percentage of non-cationic lipid(s) in the lipid nanoparticle may be greater than 5%, greater than 10%, greater than 20%, greater than 30%, or greater than 40%. The percentage of cholesterol in the lipid nanoparticle may be greater than 10%, greater than 20%, greater than 30%, or greater than 40%. The percentage of PEG-modified lipid(s) in the lipid nanoparticle may be greater than 1%, greater than 2%, greater than 5%, greater than 10%, or greater than 20%.

In certain preferred embodiments, the lipid nanoparticles for the purposes of the invention comprise at least one of the following cationic lipids: C12-200, DLin-KC2-DMA, DODAP, HGT4003, ICE, HGT5000, or HGT5001. In embodiments, the transfer vehicle comprises cholesterol. In some embodiments, the transfer vehicles comprises DMG-PEG2K. In certain embodiments, the transfer vehicle comprises one of the following lipid formulations: C12-200, DOPE, chol, DMG-PEG2K; DODAP, DOPE, cholesterol, DMG-PEG2K; HGT5000, DOPE, chol, DMG-PEG2K, HGT5001, DOPE, chol, DMG-PEG2K.

The liposomal transfer vehicles suitable for the purposes of the invention can be prepared by various techniques which are presently known in the art. Multi-lamellar vesicles (MLV) may be prepared conventional techniques, for example, by depositing a selected lipid on the inside wall of a suitable container or vessel by dissolving the lipid in an appropriate solvent, and then evaporating the solvent to leave a thin film on the inside of the vessel or by spray drying. An aqueous phase may then added to the vessel with a vortexing motion which results in the formation of MLVs. Uni-lamellar vesicles (ULV) can then be formed by homogenization, sonication or extrusion of the multi-lamellar vesicles. In addition, unilamellar vesicles can be formed by detergent removal techniques.

In certain embodiments of this invention, the compositions comprise a transfer vehicle wherein the mRNA is associated on both the surface of the transfer vehicle and encapsulated within the same transfer vehicle. For example, during preparation of such compositions, cationic liposomal transfer vehicles may associate with the mRNA through electrostatic interactions.

In certain embodiments, the compositions may be loaded with diagnostic radionuclide, fluorescent materials or other materials that are detectable in both *in vitro* and *in vivo* applications. For example, suitable diagnostic materials may include Rhodamine-dioleoylphosphatidylethanolamine (Rh-PE), Green Fluorescent Protein mRNA (GFP mRNA), *Renilla* Luciferase mRNA and Firefly Luciferase mRNA.

Selection of the appropriate size of a liposomal transfer vehicle must take into consideration the site of the target cell or tissue and to some extent the application for which the liposome is being made. In some embodiments, it may be desirable to limit transfection of the mRNA to certain cells or tissues. Alternatively, a liposomal transfer vehicle may be sized such that the dimensions of the liposome are of a sufficient diameter to limit or expressly avoid distribution into certain cells or tissues. Generally, the size of the transfer vehicle is within the range of about 25 to 250 nm, preferably less than about 250nm, 175nm, 150nm, 125nm, 100nm, 75nm, 50nm, 25nm or 10nm.

A variety of alternative methods known in the art are available for sizing of a population of liposomal transfer vehicles. One such sizing method is described in U.S. Pat. No. 4,737,323. Sonicating a liposome suspension either by bath or probe sonication produces a progressive size reduction down to small ULV less than about 0.05 microns in diameter. Homogenization is another method that relies on shearing energy to fragment large liposomes into smaller ones. In a typical homogenization procedure, MLV are recirculated through a standard emulsion homogenizer until selected liposome sizes, typically between about 0.1 and 0.5 microns, are observed. The size of the liposomal vesicles may be determined by quasi-electric light scattering (QELS) as described in Bloomfield, Ann. Rev. Biophys. Bioeng., 10:421-450 (1981). Average liposome diameter may be reduced by sonication of formed liposomes. Intermittent sonication cycles may be alternated with QELS assessment to guide efficient liposome synthesis.

### Target Cells

As used herein, the term "target cell" refers to a cell or tissue to which a composition of the invention is to be directed or targeted. The target cells are deficient in a pulmonary surfactant-associated protein .

In one embodiment, the compositions for use according to the invention facilitate a subject's endogenous production of one or more functional pulmonary surfactant-associated proteins , and in particular the production of pulmonary surfactant-associated proteins which demonstrate less immunogenicity relative to their recombinantly-prepared counterparts. Upon distribution of such compositions to the target tissues and the subsequent transfection of such target cells, the exogenous mRNA loaded into the liposomal transfer vehicle (*i.e.,* a lipid nanoparticle) is translated *in vivo* to produce a functional pulmonary surfactant-associated protein encoded by the exogenously administered mRNA. Accordingly, the compositions for use according to the present invention exploit a subject's ability to translate exogenously- or recombinantly-prepared mRNA to produce an endogenously-translated pulmonary surfactant-associated protein, and thereby produce and excrete a functional pulmonary surfactant-associated protein. The expressed or translated pulmonary surfactant-associated proteins may also be characterized by the *in vivo* inclusion of native post-translational modifications which may often be absent in recombinantly-prepared pulmonary surfactant-associated proteins, thereby further reducing the immunogenicity of the translated pulmonary surfactant-associated protein.

The administration of mRNA encoding a protein or enzyme for which the subject is deficient avoids the need to deliver the nucleic acids to specific organelles within a target cell. Rather, upon transfection of a target cell and delivery of the nucleic acids to the cytoplasm of the target cell, the mRNA contents of a transfer vehicle may be translated and a functional protein or enzyme expressed.

The present invention also enables the discriminatory targeting of target cells and tissues by both passive and active targeting means. The phenomenon of passive targeting exploits the natural distributions patterns of a transfer vehicle *in vivo* without relying upon the use of additional excipients or means to enhance recognition of the transfer vehicle by target cells.

Alternatively, the present invention enables active targeting, which involves the use of additional excipients, referred to herein as "targeting ligands" that may be bound (either covalently or non-covalently) to the transfer vehicle to encourage localization of such transfer vehicle at certain target cells or target tissues. For example, targeting may be mediated by the inclusion of one or more endogenous targeting ligands (e.g., apolipoprotein E) in or on the transfer vehicle to encourage distribution to the target cells or tissues. Recognition of the targeting ligand by the target tissues actively facilitates tissue distribution and cellular uptake of the transfer vehicle and/or its contents in the target cells and tissues. As provided herein, the composition can comprise a ligand capable of enhancing affinity of the composition to the target cell. Targeting ligands may be linked to the outer bilayer of the lipid particle during formulation or post-formulation. These methods are well known in the art. In addition, some lipid particle formulations may employ fusogenic polymers such as PEAA, hemagluttinin, other lipopeptides (see U.S. Patent Application Ser. Nos. 08/835,281, and 60/083,294) and other features useful for *in vivo* and/or intracellular delivery. In other some embodiments, the compositions demonstrate improved transfection efficacies, and/or demonstrate enhanced selectivity towards target cells or tissues of interest. Contemplated therefore are compositions which comprise one or more ligands (e.g., peptides, aptamers, oligonucleotides, a vitamin or other molecules) that are capable of enhancing the affinity of the compositions and their nucleic acid contents for the target cells or tissues. Suitable ligands may optionally be bound or linked to the surface of the transfer vehicle. In some embodiments, the targeting ligand may span the surface of a transfer vehicle or be encapsulated within the transfer vehicle. Suitable ligands and are selected based upon their physical, chemical or biological properties (e.g., selective affinity and/or recognition of target cell surface markers or features.) Cell-specific target sites and their corresponding targeting ligand can vary widely. Suitable targeting ligands are selected such that the unique characteristics of a target cell are exploited, thus allowing the composition to discriminate between target and non-target cells. For example, compositions of the invention may include surface markers (See Hillery AM, et al. "Drug Delivery and Targeting: For Pharmacists and Pharmaceutical Scientists" (2002) Taylor & Francis, Inc.) The presentation of such targeting ligands that have been conjugated to moieties present in the transfer vehicle (*i.e.,* a lipid nanoparticle) therefore facilitate recognition and uptake of the compositions of the present invention in target cells and tissues. Examples of suitable targeting ligands include one or more peptides, proteins, aptamers, vitamins and oligonucleotides.

### Application and Administration

As used herein, the term "subject" refers to any animal (e.g., a mammal), including, but not limited to, humans, non-human primates, rodents, and the like, to which the compositions and methods of the present invention are administered. Typically, the terms "subject" and "patient" are used interchangeably herein in reference to a human subject.

The compositions provided herein are suitable for the treatment of diseases or disorders relating to the deficiency of pulmonary surfactant-associated proteins that are excreted or secreted by the target cell into the surrounding extracellular fluid - see Table 1).

**Table 1. Secreted Proteins.**

| **Uniprot ID** | **Protein Name** | **Gene Name** |
|---|---|---|
| P07988 | Pulmonary surfactant-associated protein B | SFTPB |
| P11686 | Pulmonary surfactant-associated protein C | SFTPC |
| P35247 | Pulmonary surfactant-associated protein D | SFTPD |
| Q8IWL1 | Pulmonary surfactant-associated protein A2 | SFTPA2 |
| Q8IWL2 | Pulmonary surfactant-associated protein A1 | SFTPA1 |

In some embodiments, the composition is formulated in combination with one or more additional nucleic acids, carriers, targeting ligands or stabilizing reagents, or in pharmacological compositions where it is mixed with suitable excipients. For example, in one embodiment, the compositions of the invention may be prepared to deliver mRNA encoding two or more distinct proteins. Techniques for formulation and administration of drugs may be found in "Remington's Pharmaceutical Sciences," Mack Publishing Co., Easton, Pa., latest edition.

A wide range of molecules that can exert pharmaceutical or therapeutic effects can be delivered into target cells using compositions and methods of the invention. The molecules can be organic or inorganic. Organic molecules can be peptides, proteins, carbohydrates, lipids, sterols, nucleic acids (including peptide nucleic acids), or any combination thereof. A formulation for delivery into target cells can comprise more than one type of molecule, for example, two different nucleotide sequences, or a protein, an enzyme or a steroid.

The compositions for the use according to the present invention may be administered and dosed in accordance with current medical practice, taking into account the clinical condition of the subject, the site and method of administration, the scheduling of administration, the subject's age, sex, body weight and other factors relevant to clinicians of ordinary skill in the art. The "effective amount" for the purposes herein may be determined by such relevant considerations as are known to those of ordinary skill in experimental clinical research, pharmacological, clinical and medical arts. In some embodiments, the amount to be administered is effective to achieve at least some stabilization, improvement or elimination of symptoms and other indicators as are selected as appropriate measures of disease progress, regression or improvement by those of skill in the art. For example, a suitable amount and dosing regimen is one that causes at least transient protein production.

Suitable routes of pulmonary administration include intratracheal or inhaled administration. Aerosols containing compositions of the present invention can be inhaled (for nasal, tracheal, or bronchial delivery).

Also disclosed herein are lyophilized pharmaceutical compositions comprising one or more of the liposomal nanoparticles disclosed herein; the use of such lyophilized compositions as disclosed for example, in WO2012/170889. For example, lyophilized pharmaceutical compositions according to the invention may be reconstituted prior to administration or can be reconstituted *in vivo.* For example, a lyophilized pharmaceutical composition can be formulated in an appropriate dosage form administered such that the dosage form is rehydrated over time *in vivo* by the individual's bodily fluids.

Each of the publications, reference materials, accession numbers and the like referenced herein describe the background of the present disclosure.

### EXAMPLES

### Comparative example 1: Protein Production Depot via Intravenous Delivery of Polynucleotide Compositions

### Messenger RNA

Human erythropoietin (EPO) (SEQ ID NO: 3; **FIG. 3****),** human alpha-galactosidase (GLA) (SEQ ID NO: 4; **FIG. 4****),** human alpha-1 antitrypsin (A1AT) (SEQ ID NO: 5; **FIG. 5****),** and human factor IX (FIX) (SEQ ID NO: 6; **FIG. 6****)** were synthesized by *in vitro* transcription from a plasmid DNA template encoding the gene, which was followed by the addition of a 5' cap structure (Cap1) (Fechter & Brownlee, J. Gen. Virology 86:1239-1249 (2005)) and a 3' poly(A) tail of approximately 200 nucleotides in length as determined by gel electrophoresis. 5' and 3' untranslated regions were present in each mRNA product in the following examples and are defined by SEQ ID NOs: 1 and 2 **(****FIG. 1** and **FIG. 2****)** respectively.

### Lipid Nanoparticle Formulations

*Formulation 1:* Aliquots of 50 mg/mL ethanolic solutions of C12-200, DOPE, Chol and DMG-PEG2K (40:30:25:5) were mixed and diluted with ethanol to 3 mL final volume. Separately, an aqueous buffered solution (10 mM citrate/150 mM NaCl, pH 4.5) of mRNA was prepared from a 1 mg/mL stock. The lipid solution was injected rapidly into the aqueous mRNA solution and shaken to yield a final suspension in 20% ethanol. The resulting nanoparticle suspension was filtered, diafiltrated with 1x PBS (pH 7.4), concentrated and stored at 2-8°C.

*Formulation 2:* Aliquots of 50 mg/mL ethanolic solutions of DODAP, DOPE, cholesterol and DMG-PEG2K (18:56:20:6) were mixed and diluted with ethanol to 3 mL final volume. Separately, an aqueous buffered solution (10 mM citrate/150 mM NaCl, pH 4.5) of EPO mRNA was prepared from a 1 mg/mL stock. The lipid solution was injected rapidly into the aqueous mRNA solution and shaken to yield a final suspension in 20% ethanol. The resulting nanoparticle suspension was filtered, diafiltrated with 1x PBS (pH 7.4), concentrated and stored at 2-8°C. Final concentration = 1.35 mg/mL EPO mRNA (encapsulated). Zₐᵥₑ = 75.9 nm (Dv₍₅₀₎ = 57.3 nm; Dv₍₉₀₎ = 92.1 nm).

*Formulation 3*: Aliquots of 50 mg/mL ethanolic solutions of HGT4003, DOPE, cholesterol and DMG-PEG2K (50:25:20:5) were mixed and diluted with ethanol to 3 mL final volume. Separately, an aqueous buffered solution (10 mM citrate/150 mM NaCl, pH 4.5) of mRNA was prepared from a 1 mg/mL stock. The lipid solution was injected rapidly into the aqueous mRNA solution and shaken to yield a final suspension in 20% ethanol. The resulting nanoparticle suspension was filtered, diafiltrated with 1x PBS (pH 7.4), concentrated and stored at 2-8°C.

*Formulation 4*: Aliquots of 50 mg/mL ethanolic solutions of ICE, DOPE and DMG-PEG2K (70:25:5) were mixed and diluted with ethanol to 3 mL final volume. Separately, an aqueous buffered solution (10 mM citrate/150 mM NaCl, pH 4.5) of mRNA was prepared from a 1 mg/mL stock. The lipid solution was injected rapidly into the aqueous mRNA solution and shaken to yield a final suspension in 20% ethanol. The resulting nanoparticle suspension was filtered, diafiltrated with 1x PBS (pH 7.4), concentrated and stored at 2-8°C.

*Formulation 5:* Aliquots of 50 mg/mL ethanolic solutions of HGT5000, DOPE, cholesterol and DMG-PEG2K (40:20:35:5) were mixed and diluted with ethanol to 3 mL final volume. Separately, an aqueous buffered solution (10 mM citrate/150 mM NaCl, pH 4.5) of EPO mRNA was prepared from a 1 mg/mL stock. The lipid solution was injected rapidly into the aqueous mRNA solution and shaken to yield a final suspension in 20% ethanol. The resulting nanoparticle suspension was filtered, diafiltrated with 1x PBS (pH 7.4), concentrated and stored at 2-8°C. Final concentration = 1.82 mg/mL EPO mRNA (encapsulated). Zₐᵥₑ = 105.6 nm (Dv₍₅₀₎ = 53.7 nm; Dv₍₉₀₎ = 157 nm).

*Formulation 6:* Aliquots of 50 mg/mL ethanolic solutions of HGT5001, DOPE, cholesterol and DMG-PEG2K (40:20:35:5) were mixed and diluted with ethanol to 3 mL final volume. Separately, an aqueous buffered solution (10 mM citrate/150 mM NaCl, pH 4.5) of EPO mRNA was prepared from a 1 mg/mL stock. The lipid solution was injected rapidly into the aqueous mRNA solution and shaken to yield a final suspension in 20% ethanol. The resulting nanoparticle suspension was filtered, diafiltrated with 1x PBS (pH 7.4), concentrated and stored at 2-8°C.

### Analysis of protein produced via intravenously delivered mRNA-loaded nanoparticles Injection Protocol

Studies were performed using male CD-1 mice of approximately 6-8 weeks of age at the beginning of each experiment, unless otherwise indicated. Samples were introduced by a single bolus tail-vein injection of an equivalent total dose of 30-200 micrograms of encapsulated mRNA. Mice were sacrificed and perfused with saline at the designated time points.

### Isolation of organ tissues for analysis

The liver and spleen of each mouse was harvested, apportioned into three parts, and stored in either 10% neutral buffered formalin or snap-frozen and stored at -80°C for analysis.

### Isolation of serum for analysis

All animals were euthanized by CO₂ asphyxiation 48 hours post dose administration (± 5%) followed by thoracotomy and terminal cardiac blood collection. Whole blood (maximal obtainable volume) was collected via cardiac puncture on euthanized animals into serum separator tubes, allowed to clot at room temperature for at least 30 minutes, centrifuged at 22°C ± 5°C at 9300 g for 10 minutes, and the serum extracted. For interim blood collections, approximately 40-50µL of whole blood was collected via facial vein puncture or tail snip. Samples collected from non treatment animals were used as a baseline for comparison to study animals.

### Enzyme-Linked Immunosorbent Assay (ELISA) Analysis

*EPO ELISA:* Quantification of EPO protein was performed following procedures reported for human EPO ELISA kit (Quantikine IVD, R&D Systems, Catalog # Dep-00). Positive controls employed consisted of ultrapure and tissue culture grade recombinant human erythropoietin protein (R&D Systems, Catalog # 286-EP and 287-TC, respectively). Detection was monitored via absorption (450 nm) on a Molecular Device Flex Station instrument.

*GLA ELISA:* Standard ELISA procedures were followed employing sheep anti-Alpha-galactosidase G-188 IgG as the capture antibody with rabbit anti-Alpha-galactosidase TK-88 IgG as the secondary (detection) antibody (Shire Human Genetic Therapies). Horseradish peroxidase (HRP)-conjugated goat anti-rabbit IgG was used for activation of the 3,3',5,5'-tetramethylbenzidine (TMB) substrate solution. The reaction was quenched using 2N H₂SO₄ after 20 minutes. Detection was monitored via absorption (450 nm) on a Molecular Device Flex Station instrument. Untreated mouse serum and human Alpha-galactosidase protein were used as negative and positive controls, respectively.

*FIX ELISA:* Quantification of FIX protein was performed following procedures reported for human FIX ELISA kit (AssayMax, Assay Pro, Catalog # EF1009-1).

*A1AT ELISA:* Quantification of A1AT protein was performed following procedures reported for human A1AT ELISA kit (Innovative Research, Catalog #IRAPKT015).

### Western Blot Analysis

*(EPO)*: Western blot analyses were performed using an anti-hEPO antibody (R&D Systems #MAB2871) and ultrapure human EPO protein (R&D Systems #286-EP) as the control.

### Results

The work described in this example demonstrates the use of mRNA-encapsulated lipid nanoparticles as a depot source for the production of protein. Such a depot effect can be achieved in multiple sites within the body (i.e., liver, kidney, spleen, and muscle). Measurement of the desired exogenous-based protein derived from messenger RNA delivered via liposomal nanoparticles was achieved and quantified, and the secretion of protein from a depot using human erythropoietin (hEPO), human alpha-galactosidase (hGLA), human alpha-1 antitrypsin (hA1AT), and human Factor IX (hFIX) mRNA was demonstrated.

### 1A. In Vivo Human EPO Protein Production Results

The production of hEPO protein was demonstrated with various lipid nanoparticle formulations. Of four different cationic lipid systems, C12-200-based lipid nanoparticles produced the highest quantity of hEPO protein after four hours post intravenous administration as measured by ELISA (FIG. 7). This formulation *(Formulation* 1) resulted in 18.3 ug/mL hEPO protein secreted into the bloodstream. Normal hEPO protein levels in serum for human are 3.3-16.6 mIU/mL (NCCLS Document C28-P; Vol. 12, No. 2). Based on a specific activity of 120,000 IU/mg of EPO protein, that yields a quantity of 27.5-138 pg/mL hEPO protein in normal human individuals. Therefore, a single 30 ug dose of a C12-200-based cationic lipid formulation encapsulating hEPO mRNA yielded an increase in respective protein of over 100,000-fold physiological levels.

Of the lipid systems tested, the DODAP-based lipid nanoparticle formulation was the least effective. However, the observed quantity of human EPO protein derived from delivery via a DODAP-based lipid nanoparticle encapsulating EPO mRNA was 4.1 ng/mL, which is still greater than 30-fold over normal physiological levels of EPO protein (**Table 2**).

**Table 2. Raw values of secreted hEPO protein for various cationic lipid-based nanoparticle systems as measured via ELISA analysis (as depicted in FIG. 8). Doses are based on encapsulated hEPO mRNA. Values of protein are depicted as nanogram of human EPO protein per milliliter of serum. Hematocrit changes are based on comparison of pre-bleed (Day -1) and Day 10.**

| **Cationic/Ionizable Lipid Component** | **Dose of Encapsulated mRNA (ug)** | **Secreted Human EPO Protein (ng/mL)** | **Increase in Hematocrit (%)** |
|---|---|---|---|
| C12-200 | 30 | 18,306 | 15.0 |
| HGT4003 | 150 | 164 | 0.0 |
| ICE | 100 | 56.2 | 0.0 |
| DODAP | 200 | 4.1 | 0.0 |

In addition, the resulting protein was tested to determine if it was active and functioned properly. In the case of mRNA replacement therapy (MRT) employing hEPO mRNA, hematocrit changes were monitored over a ten day period for five different lipid nanoparticle formulations (**FIG. 8****, Table 2**) to evaluate protein activity. During this time period, two of the five formulations demonstrated an increase in hematocrit (≥15%), which is indicative of active hEPO protein being produced from such systems.

In another experiment, hematocrit changes were monitored over a 15-day period (**FIG. 9****, Table 3**). The lipid nanoparticle formulation (*Formulation 1*) was administered either as a single 30 µg dose, or as three smaller 10 µg doses injected on day 1, day 3 and day 5. Similarly, *Formulation 2* was administered as 3 doses of 50 µg on day 1, day 3, and day 5. C12-200 produced a significant increase in hematocrit. Overall an increase of up to ~25% change was observed, which is indicative of active human EPO protein being produced from such systems.

**Table 3 Hematocrit levels of each group over a 15 day observation period (FIG. 9). Mice were either dosed as a single injection, or three injections, every other day. N=4 mice per group.**

| **Test Article** | **Dose (µg/animal)** | **Hct Levels Mean (%) ± SEM** | | | |
|---|---|---|---|---|---|
| | | **Day -4** | **Day 7** | **Day 10** | **Day 15^{a}** |
| C12-200 | **30 (single dose)** | **50.8±1.8** | **58.3±3.3** | **62.8±1.3** | **59.9±3.3** |
| C12-200 | **30 (over 3 doses)** | **52.2±0.5** | **55.3±2.3** | **63.3±1.6** | **62.3±1.9** |
| DODAP | **150 (over 3 doses)** | **54.8±1.7** | **53.5±1.6** | **54.2±3.3** | **54.0±0.3** |

| | | | | | |
|---|---|---|---|---|---|
| Hct = hematocrit; SEM = standard error of the mean. ^{a}Blood samples were collected into non-heparinized hematocrit tubes. | | | | | |

### 1B. In Vivo Human GLA Protein Production Results

A second exogenous-based protein system was explored to demonstrate the "depot effect" when employing mRNA-loaded lipid nanoparticles. Animals were injected intravenously with a single 30 microgram dose of encapsulated human alpha-galactosidase (hGLA) mRNA using a C12-200-based lipid nanoparticle system and sacrificed after six hours *(Formulation* 1). Quantification of secreted hGLA protein was performed via ELISA. Untreated mouse serum and human Alpha-galactosidase protein were used as controls. Detection of human alpha-galactosidase protein was monitored over a 48 hour period.

Measurable levels of hGLA protein were observed throughout the time course of the experiment with a maximum level of 2.0 ug/mL hGLA protein at six hours **(****FIG. 10****). Table 4** lists the specific quantities of hGLA found in the serum. Normal activity in healthy human males has been reported to be approximately 3.05 nanomol/hr/mL. The activity for Alpha-galactosidase, a recombinant human alpha-galactosidase protein, 3.56 × 10⁶ nanomol/hr/mg. Analysis of these values yields a quantity of approximately 856 pg/mL of hGLA protein in normal healthy male individuals. The quantity of 2.0 ug/mL hGLA protein observed after six hours when dosing a hGLA mRNA-loaded lipid nanoparticle is over 2300-fold greater than normal physiological levels. Further, after 48 hours, one can still detect appreciable levels of hGLA protein (86.2 ng/mL). This level is representative of almost 100-fold greater quantities of hGLA protein over physiological amounts still present at 48 hours.

**Table 4. Raw values of secreted hGLA protein over time as measured via ELISA analysis (as depicted in FIG. 10). Values are depicted as nanogram of hGLA protein per milliliter of serum. N=4 mice per group.**

| **Time Post-Administration (hr)** | **Secreted Human GLA Protein (ng/mL)** |
|---|---|
| 6 | 2,038 |
| 12 | 1,815 |
| 24 | 414 |
| 48 | 86.2 |

In addition, the half-life of Alpha-galactosidase when administered at 0.2 mg/kg is approximately 108 minutes. Production of GLA protein via the "depot effect" when administering GLA mRNA-loaded lipid nanoparticles shows a substantial increase in blood residence time when compared to direct injection of the naked recombinant protein. As described above, significant quantities of protein are present after 48 hours.

The activity profile of the α-galactosidase protein produced from GLA mRNA-loaded lipid nanoparticles was measured as a function of 4-methylumbelliferyl-α-D-galactopyranoside (4-MU-α-gal) metabolism. As shown in **FIG. 11****,** the protein produced from these nanoparticle systems is quite active and reflective of the levels of protein available **(****FIG. 12****, Table 4).** AUC comparisons of mRNA therapy-based hGLA production versus enzyme replacement therapy (ERT) in mice and humans show a 182-fold and 30-fold increase, respectively **(Table 5).**

**Table 5. Comparison of Cₘₐₓ and AUC_{inf} values in Fabry patients post-IV dosing 0.2 mg/kg of Alpha-galactosidase (pharmacological dose) with those in mice post-IV dosing Alpha-galactosidase and GLA mRNA. ^{a} Data were from a published paper (Gregory M. Pastores et al. Safety and Pharmacokinetics of hGLA in patients with Fabry disease and end-stage renal disease. Nephrol Dial Transplant (2007) 22:1920-1925. ^{b} non-end-stage renal disease. ^{c} α-Galactosidase activity at 6 hours after dosing (the earliest time point tested in the study).**

| | Test Article | Description | Dose (mg/kg) | Cₘₐₓ (U/mL) | AUC_{inf} (hr.U/mL) | n |
|---|---|---|---|---|---|---|
| Fabry^{a} Patient | α-GAL Protein | Transplant | 0.2 | 3478 | 3683 | 11 |
| | | Dialysis | 0.2 | 3887 | 3600 | 6 |
| | | Non-ESRD^{b} | 0.2 | 3710 | 4283 | 18 |
| Mouse | α-GAL Protein (MM1) | Athymic nude | 0.04 | 3807 | 797 | 3 |
| | α-GAL Protein (MM2) | Athymic nude | 0.04 | 3705 | 602 | 3 |
| | α-GAL | | | | | |
| Mouse | mRNA | mouse | 0.95 | 5885 (C_{at 6hr})^{c} | 109428 | 6 |

The ability of mRNA encapsulated lipid nanoparticles to target organs which can act as a depot for the production of a desired protein has been demonstrated. The levels of secreted protein observed have been several orders of magnitude above normal physiological levels. This "depot effect" is repeatable. **FIG. 12** shows again that robust protein production is observed upon dosing wild type (CD-1) mice with a single 30 ug dose of hGLA mRNA-loaded in C12-200-based lipid nanoparticles *(Formulation 1*) . In this experiment, hGLA levels were evaluated over a 72 hour period. A maximum average of 4.0 ug human hGLA protein/mL serum is detected six hours post-administration. Based on a value of ~1 ng/mL hGLA protein for normal physiological levels, hGLA MRT provides roughly 4000-fold higher protein levels. As before, hGLA protein could be detected out to 48 hr post-administration **(****FIG. 12****).**

An analysis of tissues isolated from this same experiment provided insight into the distribution of hGLA protein in hGLA MRT-treated mice **(****FIG. 13****).** Supraphysiological levels of hGLA protein were detected in the liver, spleen and kidneys of all mice treated with a maximum observed between 12 and 24 hour post-administration. Detectable levels of MRT-derived protein could be observed three days after a single injection of hGLA-loaded lipid nanoparticles.

In addition, the production of hGLA upon administration of hGLA mRNA loaded C12-200 nanoparticles was shown to exhibit a dose a response in the serum **(****FIG. 14A****),** as well as in the liver **(****FIG. 14B****).** One inherent characteristic of lipid nanoparticle-mediated mRNA replacement therapy would be the pharmacokinetic profile of the respective protein produced. For example, ERT-based treatment of mice employing Alpha-galactosidase results in a plasma half-life of approximately 100 minutes. In contrast, MRT-derived alpha-galactosidase has a blood residence time of approximately 72 hrs with a peak time of 6 hours. This allows for much greater exposure for organs to participate in possible continuous uptake of the desired protein. A comparison of PK profiles is shown in **FIG. 15** and demonstrates the stark difference in clearance rates and ultimately a major shift in area under the curve (AUC) can be achieved via MRT-based treatment.

In a separate experiment, hGLA MRT was applied to a mouse disease model, hGLA KO mice (Fabry mice). A 0.33 mg/kg dose of hGLA mRNA-loaded C12-200-based lipid nanoparticles *(Formulation 1*) was administered to female KO mice as a single, intravenous injection. Substantial quantities of MRT-derived hGLA protein were produced with a peak at 6 hr (~560 ng/mL serum) which is approximately 600-fold higher than normal physiological levels. Further, hGLA protein was still detectable 72 hr post-administration **(****FIG. 16****).**

Quantification of MRT-derived GLA protein in vital organs demonstrated substantial accumulation as shown in **FIG. 17****.** A comparison of observed MRT-derived hGLA protein to reported normal physiological levels that are found in key organs is plotted (normal levels plotted as dashed lines). While levels of protein at 24 hours are higher than at 72 hours post-administration, the levels of hGLA protein detected in the liver, kidney, spleen and hearts of the treated Fabry mice are equivalent to wild type levels. For example, 3.1 ng hGLA protein/mg tissue were found in the kidneys of treated mice 3 days after a single MRT treatment.

In a subsequent experiment, a comparison of ERT-based Alpha-galactosidase treatment versus hGLA MRT-based treatment of male Fabry KO mice was conducted. A single, intravenous dose of 1.0 mg/kg was given for each therapy and the mice were sacrificed one week post-administration. Serum levels of hGLA protein were monitored at 6 hr and 1 week post-injection. Liver, kidney, spleen, and heart were analyzed for hGLA protein accumulation one week post-administration. In addition to the biodistribution analyses, a measure of efficacy was determined via measurement of globotrioasylceramide (Gb3) and lyso-Gb3 reductions in the kidney and heart. **FIG. 18** shows the serum levels of hGLA protein after treatment of either Alpha-galactosidase or GLA mRNA loaded lipid nanoparticles *(Formulation 1*) in male Fabry mice. Serum samples were analyzed at 6 hr and 1 week post-administration. A robust signal was detected for MRT-treated mice after 6 hours, with hGLA protein serum levels of ~4.0 ug/mL. In contrast, there was no detectable Alpha-galactosidase remaining in the bloodstream at this time.

The Fabry mice in this experiment were sacrificed one week after the initial injection and the organs were harvested and analyzed (liver, kidney, spleen, heart). **FIG. 19** shows a comparison of human GLA protein found in each respective organ after either hGLA MRT or Alpha-galactosidase ERT treatment. Levels correspond to hGLA present one week post-administration. hGLA protein was detected in all organs analyzed. For example, MRT-treated mice resulted in hGLA protein accumulation in the kidney of 2.42 ng hGLA protein/mg protein, while Alpha-galactosidase-treated mice had only residual levels (0.37 ng/mg protein). This corresponds to a ~6.5-fold higher level of hGLA protein when treated via hGLA MRT. Upon analysis of the heart, 11.5 ng hGLA protein/mg protein was found for the MRT-treated cohort as compared to only 1.0 ng/mg protein Alpha-galactosidase. This corresponds to an ~11-fold higher accumulation in the heart for hGLA MRT-treated mice over ERT-based therapies.

In addition to the biodistribution analyses conducted, evaluations of efficacy were determined via measurement of globotrioasylceramide (Gb3) and lyso-Gb3 levels in key organs. A direct comparison of Gb3 reduction after a single, intravenous 1.0 mg/kg GLA MRT treatment as compared to a Alpha-galactosidase ERT-based therapy of an equivalent dose yielded a sizeable difference in levels of Gb3 in the kidneys as well as heart. For example, Gb3 levels for GLA MRT versus Alpha-galactosidase yielded reductions of 60.2% vs. 26.8%, respectively **(****FIG. 20****).** Further, Gb3 levels in the heart were reduced by 92.1% vs. 66.9% for MRT and Alpha-galactosidase, respectively **(****FIG. 21****).**

A second relevant biomarker for measurement of efficacy is lyso-Gb3. GLA MRT reduced lyso-Gb3 more efficiently than Alpha-galactosidase as well in the kidneys and heart **(****FIG. 20** and **FIG. 21****,** respectively). In particular, MRT-treated Fabry mice demonstrated reductions of lyso-Gb3 of 86.1% and 87.9% in the kidneys and heart as compared to Alpha-galactosidase-treated mice yielding a decrease of 47.8% and 61.3%, respectively.

The results with for hGLA in C12-200 based lipid nanoparticles extend to other lipid nanoparticle formulations. For example, hGLA mRNA loaded into HGT4003 *(Formulation 3*) or HGT5000- based *(Formulation 5*) lipid nanoparticles administered as a single dose IV result in production of hGLA at 24 hours post administration **(****FIG. 22****).** The production of hGLA exhibited a dose response. Similarly, hGLA production was observed at 6 hours and 24 hours after administration of hGLA mRNA loaded into HGT5001- based *(Formulation 6*) lipid nanoparticles administered as a single dose IV. hGLA production was observed in the serum **(****FIG. 23A****),** as well as in organs **(****FIG. 23B****).**

Overall, mRNA replacement therapy applied as a depot for protein production produces large quantities of active, functionally therapeutic protein at supraphysiological levels. This method has been demonstrated to yield a sustained circulation half-life of the desired protein and this MRT-derived protein is highly efficacious for therapy as demonstrated with alpha-galactosidase enzyme in Fabry mice.

### 1C. In Vivo Human FIX Protein Production Results

Studies were performed administering Factor IX (FIX) mRNA-loaded lipid nanoparticles in wild type mice (CD-1) and determining FIX protein that is secreted into the bloodstream. Upon intravenous injection of a single dose of 30 ug C12-200-based (C12-200:DOPE:Chol:PEG at a ratio of 40:30:25:5) FIX mRNA-loaded lipid nanoparticles (dose based on encapsulated mRNA) *(Formulation 1*), a robust protein production was observed **(****FIG. 24****).**

A pharmacokinetic analysis over 72 hours showed MRT-derived FIX protein could be detected at all time points tested **(****FIG. 24****).** The peak serum concentration was observed at 24 hr post-injection with a value of ~3 ug (2995±738 ng/mL) FIX protein/mL serum. This represents another successful example of the depot effect.

### ID. In Vivo Human A1AT Protein Production Results

Studies were performed administering alpha-1-antitrypsin (A1AT) mRNA-loaded lipid nanoparticles in wild type mice (CD-1) and determining A1AT protein that is secreted into the bloodstream. Upon intravenous injection of a single dose of 30 ug C12-200-based A1AT mRNA-loaded lipid nanoparticles (dose based on encapsulated mRNA) *(Formulation 1*), a robust protein production was observed **(****FIG. 25****).**

As depicted in **FIG. 25****,** detectable levels of human A1AT protein derived from A1AT MRT could be observed over a 24 hour time period post-administration. A maximum serum level of ~48 ug A1AT protein/mL serum was detected 12 hours after injection.

### Comparataive example 2: Protein Production Depot via Pulmonary Delivery of Polynucleotide Compositions

### Injection Protocol

All studies were performed using female CD-1 or BALB/C mice of approximately 7-10 weeks of age at the beginning of each experiment. Test articles were introduced via a single intratracheal aerosolized administration. Mice were sacrificed and perfused with saline at the designated time points. The lungs of each mouse were harvested, apportioned into two parts, and stored in either 10% neutral buffered formalin or snap-frozen and stored at -80°C for analysis. Serum was isolated as described in Example 1. *EPO ELISA :* as described in Example 1.

### Results

The depot effect can be achieved via pulmonary delivery (e.g. intranasal, intratracheal, nebulization). Measurement of the desired exogenous-based protein derived from messenger RNA delivered via nanoparticle systems was achieved and quantified.

The production of human EPO protein via hEPO mRNA-loaded lipid nanoparticles was tested in CD-1 mice via a single intratracheal administration (MicroSprayer^{®}). Several formulations were tested using various cationic lipids (*Formulations 1, 5, 6*). All formulations resulted in high encapsulation of human EPO mRNA. Upon administration, animals were sacrificed six hours post-administration and the lungs as well as serum were harvested.

Human EPO protein was detected at the site of administration (lungs) upon treatment via aerosol delivery. Analysis of the serum six hours post-administration showed detectable amounts of hEPO protein in circulation. These data (shown in **FIG. 26****)** demonstrate the ability of the lung to act as a "depot" for the production (and secretion) of hEPO protein.

## Claims

1. A composition for use in a method of treating a disease in a subject that is due to a deficiency of a pulmonary surfactant-associated protein, wherein the pulmonary surfactant-associated protein is selected from SFTPA1, SFTPA2, SFTPB, SFTPC, and SFTPD, wherein said method comprises pulmonary administration of the composition to the subject, said composition comprising:
(a) at least one mRNA molecule at least a portion of which encodes the pulmonary surfactant-associated protein; and
(b) a transfer vehicle comprising a lipid nanoparticle, wherein the lipid nanoparticle comprises one or more cationic lipids, one or more non-cationic lipids and one or more PEG-modified lipids.

2. The composition for use according to claim 1, wherein the RNA molecule comprises:
(a) a modification of the 5' untranslated region of said RNA molecule, optionally wherein said modification comprises the inclusion of a Cap1 structure; and/or
(b) a modification of the 3' untranslated region of said RNA molecule, optionally wherein said modification of the 3' untranslated region comprises the inclusion of a poly A tail.

3. The composition for use according to claim 1 or 2, wherein the lipid nanoparticle comprises a cationic lipid selected from: 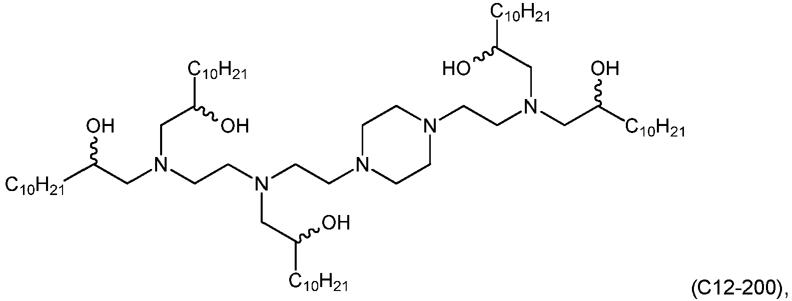
2,2-Dilinoley1-4-dimethylaminoethy1-[1,3]-dioxolane (XTC),
((6Z,9Z,28Z,3IZ)-heptatriaconta-6,9,28,31-tetraen-19-yl 4-(dimethylamino) butanoate (MC3),
4,7,13-tris(3-oxo-3-(undecylamino)propyl)-N1,N16-diundecyl-4,7,10,13-tetraazahexadecane-1,16-diamide (NC98-5), 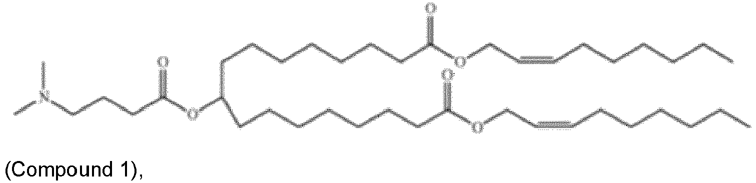 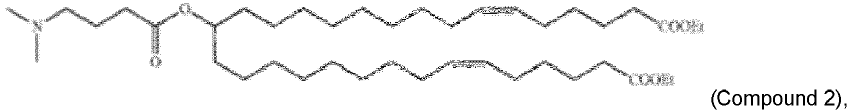 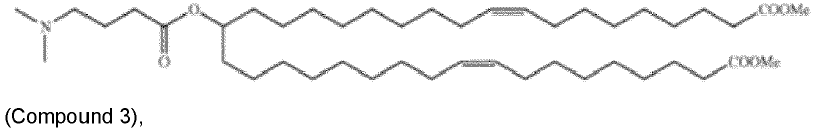
1,2-dilinoleyloxy-N,N-dimethyl-3-aminopropane (DLinDMA),
(15Z,18Z)-N,N-dimethyl-6-((9Z,12Z)-octadeca-9,12-dien-1-yl)tetracosa-4,15,18-trien-1-amine (HGT5001), *cis* or *trans,*
(15Z,18Z)-N,N-dimethyl-6-(9Z,12Z)-octadeca-9,12-dien-1-yl)tetracosa-15,18-dien-1-amine (HGT5000),
2-((2,3-Bis((9Z,12Z)-octadeca-9,12-dien-1-yloxy)propyl)disulfanyl)-N,N-dimethylethanamine (HGT4003),
2-(2,2-di((9Z,12Z)-octadeca-9,12-dien-1-yl)-1,3-dioxolan-4-yl)-N,N-dimethylethanamine (DLinKC2DMA),
(3aR,5s,6aS)-N,N-dimethyl-2,2-di((9Z,12Z)-octadeca-9,12-dienyl)tetrahydro-3aH-cyclopenta[d] [1 ,3]dioxol-5-amine) (ALNY100), and
(3S, 10R, 13R, 17R)-10, 13-dimethyl-17-((R)-6-methylheptan-2-yl)-2, 3, 4, 7, 8, 9, 10, 11, 12, 13, 14, 15, 16, 17-tetradecahydro-1H-cyclopenta[a]phenanthren-3-yl 3-(1H-imidazol-4-yl)propanoate (ICE).

4. The composition for use according to claim 1 or 2, wherein the lipid nanoparticle comprises DLinKC2DMA, cholesterol (CHOL), dioleoylphosphatidylethanolamine (DOPE), and DMG-PEG-2000.

5. The composition for use according to claims 1 or 2, wherein the lipid nanoparticle comprises C12-200, DOPE, CHOL, and DMG-PEG-2000.

6. The composition for use according to any one of claims 1-5, wherein the lipid nanoparticle comprises a cleavable lipid.

7. The composition for use according to any one of claims 1-6, wherein the at least one mRNA is (a) associated on both the surface of the lipid nanoparticle and encapsulated within the same lipid nanoparticle, or (b) is encapsulated within the lipid nanoparticle.

8. The composition for use according to any one of claims 1-7, wherein the transfer vehicle comprises a cholesterol-based cationic lipid, optionally wherein the cholesterol-based cationic lipid is ICE.

## Patentansprüche

1. Zusammensetzung zur Verwendung in einem Verfahren zur Behandlung einer Erkrankung bei einem Individuum, die auf einen Mangel an einem pulmonalen tensid-assoziierten Protein zurückzuführen ist, wobei das pulmonale tensid-assoziierte Protein aus SFTPA1, SFTPA2, SFTPB, SFTPC, und SFTPD ausgewählt ist, wobei das Verfahren eine pulmonale Verabreichung der Zusammensetzung an das Individuum umfasst, wobei die Zusammensetzung Folgendes umfasst:
(a) mindestens ein mRNA-Molekül, wobei zumindest ein Abschnitt desselben für das pulmonale tensid-assoziierte Protein codiert; und
(b) einen Überführungsträger, der einen Lipidnanopartikel umfasst, wobei der Lipidnanopartikel ein oder mehrere kationische Lipide, ein oder mehrere nicht-kationische Lipide und ein oder mehrere PEGmodifizierte Lipide umfasst.

2. Zusammensetzung zur Verwendung nach Anspruch 1, wobei das RNA-Molekül Folgendes umfasst:
(a) eine Modifizierung der nicht-translatierten 5'-Region des RNA-Moleküls, wobei die Modifizierung möglicherweise das Einführen einer Cap1-Struktur umfasst; und/oder
(b) eine Modifizierung der nicht-translatierten 3'-Region des RNA-Moleküls, wobei die Modifizierung der nicht-translatierten 3'-Region möglicherweise das Einführen eines Poly-A-Schwanzes umfasst.

3. Zusammensetzung zur Verwendung nach Anspruch 1 oder 2, wobei der Lipidnanopartikel ein kationisches Lipid umfasst, das aus Folgendem ausgewählt ist: 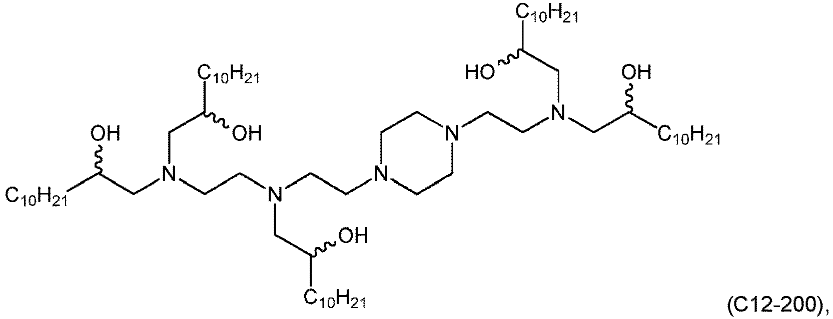
2,2-Dilinoleyl-4-dimethylaminoethyl[1,3]-dioxolan (XTC),
((6Z,9Z,28Z,31Z)-Heptatriaconta-6,9,28,31-tetraen-19-yl-4-(dimethylamino)butanoat (MC3),
4,7,13-tris-(3-Oxo-3-(undecylamino)propyl)-N1,N16-diundecyl-4,7,10,13-tetraazahexadecan-1,16-diamid (NC98-5), 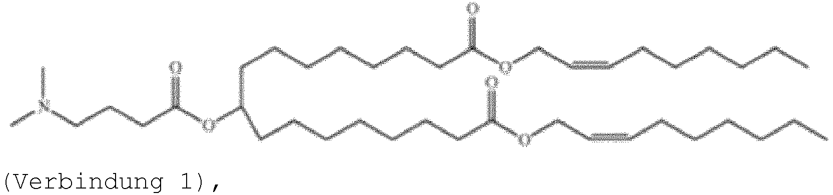 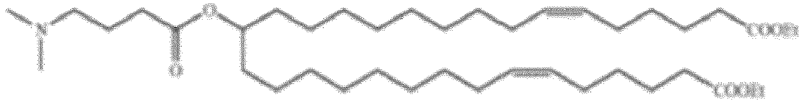 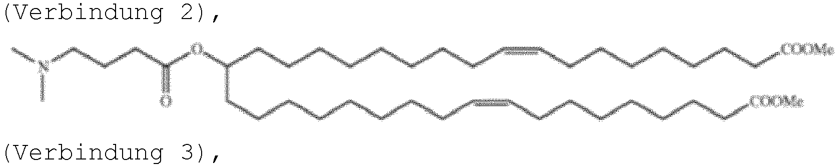
1,2-Dilinoleyloxy-N,N-dimethyl-3-aminopropan (DLinDMA), (15Z,18Z)-N,N-Dimethyl-6-(9Z,12Z)-octadeca-9,12-dien-1-yl)tetracosa-4,15,18-trien-1-amin (HGT5001), *cis* oder *trans,*
(15Z,18Z)-N,N-Dimethyl-6-(9Z,12Z)-octadeca-9,12-dien-1-yl)tetracosa-15,18-dien-1-amin (HGT5000),
2-((2,3-bis-((9Z,12Z)-Octadeca-9,12-dien-1-yloxy)propyl)disulfanyl)-N,N-dimethylethanamin (HGT4003),
2-(2,2-Di-((9Z,12Z)-octadeca-9,12-dien-1-yl)-1,3-dioxolan-4-yl)-N,N-dimethylethanamin (DLinKC2DMA), (3aR,5s,6aS)-N,N-Dimethyl-2,2-di((9Z,12Z)-octadeca-9,12-dienyl)tetrahydro-3aH-cyclopenta[d][1,3]dioxol-5-amin) (ALNYL00), und
(3S,10R,13R,17R)-10,13-Dimethyl-17-((R)-6-methylheptan-2-yl)-2,3,4,7,8,9,10,11,12,13,14,15,16,17-tetradecahydro-1H-cyclopenta[a]phenanthren-3-yl-3-(1H-imidazol-4-yl)propanoat (ICE).

4. Zusammensetzung zur Verwendung nach Anspruch 1 oder 2, wobei der Lipidnanopartikel DLinKC2DMA, Cholesterin (CHOL), Dioleoylphosphatidylethanolamin (DOPE) und DMG-PEG-2000 umfasst.

5. Zusammensetzung zur Verwendung nach Anspruch 1 oder 2, wobei der Lipidnanopartikel C12-200, DOPE, CHOL und DMG-PEG-2000 umfasst.

6. Zusammensetzung zur Verwendung nach Anspruch 1 bis 5, wobei der Lipidnanopartikel ein spaltbares Lipid umfasst.

7. Zusammensetzung zur Verwendung nach einem beliebigen der Ansprüche 1 bis 6, wobei die mindestens eine mRNA (a) auf beiden Seiten der Oberfläche des Lipidnanopartikels angelagert ist und in demselben Lipidnanopartikel verkapselt ist, oder (b) in dem Lipidnanopartikel verkapselt ist.

8. Zusammensetzung zur Verwendung nach einem beliebigen der Ansprüche 1 bis 7, wobei der Überführungsträger ein cholesterinbasiertes kationisches Lipid umfasst, wobei es sich bei dem cholesterinbasierten kationischen Lipid um ICE handelt.

## Revendications

1. Composition pour une utilisation dans un procédé de traitement d'une maladie chez un sujet qui est due à une déficience d'une protéine associée à un tensioactif pulmonaire, la protéine associée à un tensioactif pulmonaire étant choisie parmi SFTPA1, SFTPA2, SFTPB, SFTPC et SFTPD, ledit procédé comprenant une administration pulmonaire de la composition au sujet, ladite composition comprenant :
(a) au moins une molécule d'ARNm, au moins une partie de laquelle codant pour la protéine associée à un tensioactif pulmonaire ; et
(b) un véhicule de transfert comprenant une nanoparticule lipidique, la nanoparticule lipidique comprenant un ou plusieurs lipides cationiques, un ou plusieurs lipides non cationiques et un ou plusieurs lipides modifiés par PEG.

2. Composition pour une utilisation selon la revendication 1, la molécule d'ARN comprenant :
(a) une modification de la région non traduite 5' de ladite molécule d'ARN, éventuellement ladite modification comprenant l'inclusion d'une structure Cap1 ; et/ou
(b) une modification de la région non traduite 3' de ladite molécule d'ARN, éventuellement ladite modification de la région non traduite 3' comprenant l'inclusion d'une queue poly A.

3. Composition pour une utilisation selon la revendication 1 ou 2, la nanoparticule lipidique comprenant un lipide cationique choisi parmi : 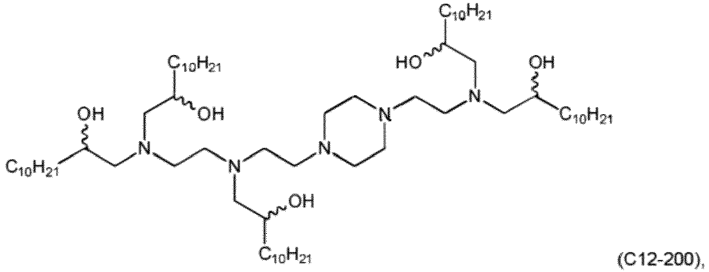
2,2-Dilinoléyl-4-diméthylaminoéthyl-[1,3]-dioxolane (XTC),
butanoate de ((6Z,9Z,28Z,31Z)-heptatriaconta-6,9,28,31-tétraén-19-yl 4-(diméthylamino) (MC3), 4,7,13-tris(3-oxo-3-(undécylamino)propyl)-N1,N16-diundécyl-4,7,10,13-tétraazahexadécane-1,16-diamide (NC98-5), 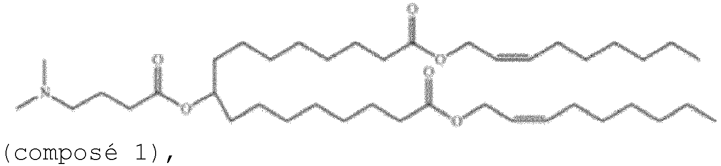 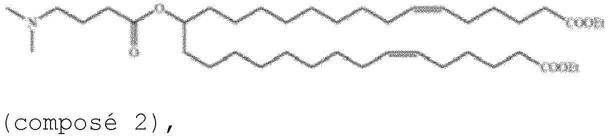 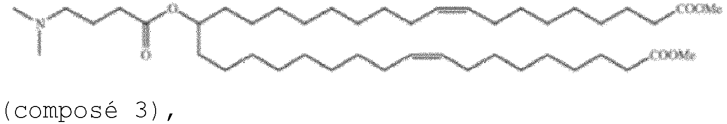
1,2-dilinoléyloxy-N,N-diméthyl-3-aminopropane (DLinDMA),
(15Z, 18Z)-N,N-diméthyl-6-((9Z,12Z)-octadéca-9,12-dién-1-yl)tétracosa-4,15,18-trién-1-amine (HGT5001), cis ou trans,
(15Z,18Z)-N,N-diméthyl-6-(9Z,12Z)-octadéca-9,12-dién-1-yl)tétracosa-15,18-dién-1-amine (HGT5000), 2-((2,3-Bis((9Z,12Z)-octadéca-9,12-dién-1-yloxy)propyl)disulfanyl)-N,N-diméthyléthanamine (HGT4003),
2-(2,2-di((9Z,12Z)-octadéca-9,12-dién-1-yl)-1,3-dioxolan-4-yl)-N,N-diméthyléthanamine (DLinKC2DMA),
(3aR,5s,6aS)-N,N-diméthyl-2,2-di((9Z,12Z)-octadéca-9,12-diényl)tétrahydro-3aH-cyclopenta[d][1,3]dioxol-5-amine) (ALNYL00), et
3-(1H-imidazol-4-yl)propanoate de (3S,10R,13R,17R)-10,13-diméthyl-17-((R)-6-méthylheptan-2-yl)-2,3,4,7,8,9,10,11,12,13,14,15,16,17-tétradécahydro-1H-cyclopenta[a]phénanthrén-3-yle (ICE) .

4. Composition pour une utilisation selon la revendication 1 ou 2, la nanoparticule lipidique comprenant DLinKC2DMA, cholestérol (CHOL), dioléoylphosphatidyléthanolamine (DOPE), et DMG-PEG-2000.

5. Composition pour une utilisation selon la revendication 1 ou 2, la nanoparticule lipidique comprenant C12-200, DOPE, CHOL, et DMG-PEG-2000.

6. Composition pour une utilisation selon l'une quelconque des revendications 1 à 5, la nanoparticule lipidique comprenant un lipide clivable.

7. Composition pour une utilisation selon l'une quelconque des revendications 1 à 6, l'au moins un ARNm étant (a) associé sur à la fois la surface de la nanoparticule lipidique et encapsulé dans la même nanoparticule lipidique ou (b) étant encapsulé dans la nanoparticule lipidique.

8. Composition pour une utilisation selon l'une quelconque des revendications 1 à 7, le véhicule de transfert comprenant un lipide cationique à base de cholestérol, éventuellement, le lipide cationique à base de cholestérol étant ICE.
